# FASCICULE DE BREVET EUROPEEN

(11) **EP 1 234 041 B1**
(45) Date de publication et mention de la délivrance du brevet: **09.07.2008**
(21) Numéro de dépôt: 00985364.9
(22) Date de dépôt: 30.11.2000
(51) Int. Cl.: C12N 15/52, C12N 15/62, C07K 14/395, C12Q 1/68, C12N 15/63, C12N 15/81, A61P 1/00

(54) **PROCEDES DE CRIBLAGE CELLULAIRE DE COMPOSES APTES A MODULER L'ACTIVITE DES COMPLEXES UBIQUITINE-LIGASES SCF ET LEURS APPLICATIONS**
ZELLULÄRES TESTVERFAHREN FÜR ZUSAMMENSETZUNGEN, DIE DIE AKTIVITÄT DES UBIQUITIN-LIGASE SCF KOMPLEXES BEEINFLUSSEN UND DESSEN ANWENDUNGEN
METHODS FOR CELL SCREENING OF COMPOUNDS CAPABLE OF MODULATING THE ACTIVITY OF UBIQUITIN-LIGASE SCF COMPLEXES AND THEIR USES

(30) Priorité: 01.12.1999 FR 9915138
(43) Date de publication de la demande: 28.08.2002
(73) Titulaire: CENTRE NATIONAL DE LA RECHERCHE SCIENTIFIQUE (CNRS), 75794 Paris Cedex 16 (FR)
(72) Inventeur: THOMAS, Dominique, F-91190 Gif-sur-Yvette (FR); BARBEY, Régine, F-91140 Villebon-sur-Yvette (FR); ROUILLON, Astrid, F-92160 Antony (FR); KERJAN, Yolande, F-92260 Fontenay-aux-Roses (FR)
(74) Mandataire: Vialle-Presles, Marie José
(86) Numéro de dépôt international: PCT/FR2000/003342
(87) Numéro de publication internationale: WO 2001/040499

(56) Documents cités:
- WO-A-00/22110
- WO-A-97/12962
- WO-A-99/04033
- WO-A-99/18989
- WO-A-99/38969
- US-A- 5 932 425
- PATTON E E ET AL: "Combinatorial control in ubiquitin-dependent proteolysis: don't Skp the F-box hypothesis" TRENDS IN GENETICS,NL,ELSEVIER SCIENCE PUBLISHERS B.V. AMSTERDAM, vol. 14, no. 6, 1 juin 1998 (1998-06-01), pages 236-243, XP004121083 ISSN: 0168-9525 cité dans la demande
- THOMAS AND SURDIN-KERJAN: "METABOLISM OF SULFUR AMINO ACIDS IN SACCHAROMYCES CEREVISIAE" MICROBIOL. AND MOL. BIOL., vol. 61, no. 4, décembre 1997 (1997-12), pages 503-532, XP002143735 cité dans la demande
- PATTON ET AL.: "Cdc53 IS A SCAFFOLD PROTEIN FOR MULTIPLE Cdc34/Skp1/F-box PROTEIN COMPLEXES THAT REGULATE CELL DIVISION AND METHIONINE BIOSYNTHESIS IN YEAST" GENES DEV., vol. 12, 1998, pages 692-705, XP002143736 cité dans la demande
- KOLODZIEJ P A ET AL: "EPITOPE TAGGING AND PROTEIN SURVEILLANCE" METHODS IN ENZYMOLOGY,US,ACADEMIC PRESS INC, SAN DIEGO, CA, vol. 194, 1991, pages 508-519, XP002057283 ISSN: 0076-6879
- CORMACK B P ET AL: "FACS-optimized mutants of the green fluorescent protein (GFP)" GENE,NL,ELSEVIER BIOMEDICAL PRESS. AMSTERDAM, vol. 173, no. 1, 1996, pages 33-38, XP004042850 ISSN: 0378-1119
- CIECHANOVER A ET AL: "THE UBIQUITIN-MEDIATED PROTEOLYTIC SYSTEM: INVOLVEMENT OF MOLECULAR CHAPERONES, DEGRADATION OF ONCOPROTEINS, AND ACTIVATION OF TRANSCRIPTIONAL REGULATORS" COLD SPRING HARBOR SYMPOSIA ON QUANTITATIVE BIOLOGY,US,NEW YORK, NY, vol. 60, 1995, pages 491-501, XP002069094
- SPELLMAN ET AL.: "COMPREHENSIVE IDENTIFICATION OF CELL CYCLE-REGULATED GENES OF THE YEAST SACCHAROMYCES CEREVISIAE BY MICROARRAY HYBRIDIZATION" MOL.BIOL.CELL, vol. 9, décembre 1998 (1998-12), pages 3273-3297, XP002143737
- PATTON ET AL.: "SCF(Met30)-MEDIATED CONTROL OF THE TRANSCRIPTIONAL ACTIVATOR Met4p IS REQUIRED FOR THE G1-S TRANSITION" EMBO J., vol. 19, no. 7, avril 2000 (2000-04), pages 1613-1624, XP002143738
- ROUILLON ET AL.: "FEEDBACK-REGULATED DEGRADATION OF THE TRANSCRIPTIONAL ACTIVATOR Met4 IS TRIGGERED BY THE SCF(Met30) COMPLEX" EMBO J., vol. 19, no. 2, janvier 2000 (2000-01), pages 282-294, XP002143739

## Description

La présente invention a pour objet des procédés de criblage cellulaire de composés aptes à moduler l'activité des complexes ubiquitine-ligases SCF et leurs applications.

L'existence de la dégradation contrôlée des protéines est connue depuis plus de trente ans mais les mécanismes moléculaires exacts, impliqués dans ce processus, n'ont été décrits que ces dernières années.

Dans les cellules eucaryotes, la voie principale de dégradation sélective des protéines en dehors des lysosomes, implique une cascade de réactions qui conduisent, dans un premier temps, au marquage des protéines à détruire par un polypeptide, constitué de 76 acides aminés, appelé ubiquitine. L'ajout de plusieurs molécules d'ubiquitine, cible alors la protéine ainsi modifiée vers le protéasome, où elle est détruite. On parle de la voie ubiquitine-protéasome.

La dégradation d'une protéine étant par essence un processus irréversible, le système ubiquitine-protéasome est recruté par les nombreuses régulations et voies de signalisation qui ont pour but de modifier à long terme les processus cellulaires, notamment les voies de développement, les régulations du cycle cellulaire et les réponses à la présence d'agents pathogènes. Il est clair que la compatibilité de tels processus avec la vie cellulaire normale nécessite un contrôle étroit de la nature des protéines à détruire.

Cette nécessaire sélectivité des processus de dégradation des protéines est assurée par les enzymes qui catalysent l'ajout des molécules d'ubiquitine et qui sont connues sous le nom générique d'ubiquitine-ligases.

Le protéasome est un complexe protéique, composé de plusieurs sous-unités, qui reconnaît les protéines lorsqu'elles ont été modifiées par l'attachement d'ubiquitine sur leurs résidus lysine. Cette ubiquitinylation, préalable à la reconnaissance des protéines cibles par le protéasome, fait intervenir au moins trois complexes enzymatiques, appelés E1, E2 et E3. E1 catalyse l'activation de l'ubiquitine par formation d'un thioester entre lui-même et l'ubiquitine, laquelle est ensuite transférée sur l'enzyme E2, enzyme de conjugaison. Finalement, l'ubiquitine-ligase E3, facilite la reconnaissance de la cible par E2 ou transfère directement l'ubiquitine de E2 sur le substrat (Hochstrasser M. et al., (1996), Annu. Rev. Genet, 30, 405-439).

Récemment il a été montré qu'un facteur de conjugaison supplémentaire, appelé E4, était nécessaire (Koegl M. et al., (1999), Cell, 96, 635-644).

Alors que le facteur E 1 est commun à toutes les voies de dégradation et ne sert qu'à activer l'ubiquitine, la sélectivité du complexe responsable de l'ubiquitinylation est assurée par l'ubiquitine-ligase E3, qui interagit à la fois avec E2 et avec le substrat (Hershko A. et al., (1983), J. Biol. Chem., 258, 8206-8214).

On distingue deux groupes d'ubiquitine-ligases :
- des protéines E3 isolées, en particulier la famille des protéines HECT (« *Homologous to E6-AP Carboxyl-Terminus* ») qui possèdent un domaine carboxy-terminal homologue à celui de la protéine E6-AP humaine, impliqué dans la formation d'un intermédiaire catalytique avec l'ubiquitine, et
- des complexes E3, parmi lesquels la famille des complexes ubiquitine-ligases SCF (S : Skpl ; C : Cdc53 ou culline ; F : protéine contenant une boîte F), est la plus diversifiée. Les ligases SCF ont été découvertes chez la levure *Saccharomyces cerevisiae* (Feldman R. M. R. et al., (1997), Cell, 91, 221-230; Skowyra D. et al., (1999), Science, 284, 662-665 ; Patton E. E. et al., (1998), Genes Dev., 12, 692-705) et il a été montré récemment qu'elles existent en fait dans tous les organismes eucaryotes, des champignons aux mammifères (Koepp D. M. et al., (1999), Cell, 97, 431-434). Les complexes SCF comprennent au moins trois sous-unités communes, la protéine Skpl, une protéine de la famille des cullines (Cdc53 chez la levure et la culline 1 chez l'homme) et la protéine Hrt1 (Rbx1 ou Rox1). Elles comprennent également des sous-unités réceptrices modulaires, qui confèrent la spécificité vis-à-vis du substrat, et qui sont des protéines contenant une boîte F (Skowyra D. *et al.,* (1999) référence citée ; Patton E. E. et al., (1998), Trends Genet., 14, 236-243). Le domaine F est un motif dégénéré d'environ 40 acides aminés, qui permet à la protéine qui le contient, d'interagir spécifiquement avec Skpl (Bai C. et al., (1996), Cell, 86, 263-274). Les complexes SCF sont très étroitement associés à une enzyme E2 particulière, appelée Cdc34, qui reconnaît un site de fixation indépendant sur Cdc53 (Patton E. E. et al., (1998), Genes dev référence citée).

A l'heure actuelle, bien que plus de 15 protéines contenant des boîtes F aient été identifiées grâce à l'homologie de leur séquence dans le génome de la levure, seulement trois complexes ont été décrits et caractérisés dans cet organisme, à savoir : SCF^{cdc4}, SCF ^{Grrl} et SCF^{Met30}. Chacun de ces complexes a pour cible un ou plusieurs substrat spécifique qui sera dégradé après ubiquitinylation ; ainsi SCF^{Cdc4} a pour cible les inhibiteurs de CDK *(cycline-dependent kinases),* Siclp et Farlp, SCF^{Grr1} a pour cible les cyclines Gl, Cln1/Cln2 et SCF^{Met30} a pour cible l'inhibiteur de CDK, Swep1 (Koepp D. M. *et al.,* (1999), référence citée). Les inventeurs ont montré que le complexe SCF^{Met30} joue également un rôle dans la régulation négative du métabolisme des acides aminés soufrés (Thomas D. et al., (1995), Mol. Cell. Biol., 15, 6526-6534).

Des homologues du complexe SCF^{Met30} ont été récemment découverts dans d'autres organismes. Ainsi, on a identifié, chez la drosophile, un gène Slimb *(Supernumerary Limbs)* codant pour des protéines à boîte F, qui ont pour cible la protéine inhibitrice IκB, le coactivateur transcriptionnel β-caténine/Armadillo (β-cat/Arm) ou la protéine régulatrice *Cubitus interruptus* (Ci) (Spencer E. et al., (1999), Genes. Dev., 13, 284-294).

Chez l'homme, un complexe homologue du complexe SCF ^{Met30}, le complexe SCF^{β-TrCP} a également été identifié. La protéine β-TrCP (*β-transducing repeat containing protein)* a été décrite, pour la première fois, dans le cadre d'une infection par le virus à VIH-1. C'est une protéine à boîte F, induite par la protéine virale Vpu, (Margottin F. et al., (1998). Mol. Cell., 1, 565-574), qui intervient dans la dégradation des récepteurs cellulaires CD4 présents à la surface des cellules infectées, et est nécessaire à l'obtention de particules virales HIV infectieuses. Des études complémentaires ont permis de montrer qu'en l'absence d'une infection par le virus à VIH-1, la β-TrCP permet la reconnaissance spécifique et la destruction de cibles telles que IκBα, inhibiteur du facteur de transcription ubiquitaire NFκB, qui régule directement la réponse immune et inflammatoire, la β-caténine, protéine de la voie Wg/Wnt, dont la stabilisation anormale conduit à l'activation de la transcription des gènes oncogéniques et qui est impliquée dans plusieurs types de cancers (Hart M. et al., (1999), Curr. Biol., 9, 207-210 ; Kroll et al., (1999), J. Biol. Chem., 274, 7941-7945).

Plus récemment, une protéine homologue de la β-TrCP, la protéine FWD1 a été identifiée chez les souris (Hatakeyama S. et al., (1999), Proc. Natl. Acad. Sci. USA, 96, 3859-3863).

Les complexes SCF constituent en fait, les prototypes d'une super-famille encore plus large d'ubiquitine-ligases, comprenant également les complexes APC *(anaphase promoting complex*)*,* qui contrôlent la division cellulaire, et les complexes VCB *(VHL-ElonginC*/*ElonginB),* en particulier ceux qui interviennent dans certaines formes rares de cancers héréditaires et ceux qui contiennent une boîte SOCS *(suppressor of cytokine signalling).*

Dans la plupart des voies de signalisation qui font intervenir le protéasome, la cascade de signalisation qui suit, est conservée :
A. activation ou inhibition d'une protéine kinase, sous l'influence d'un stimulus extracellulaire,
B. phosphorylation (ou déphosphorylation) de la protéine cible,
C. reconnaissance et ubiquitinylation de la protéine cible phosphorylée par le complexe SCF, et
D. ciblage de la protéine ubiquitinylée vers le protéasome où elle est finalement dégradée. La protéine déphosphorylée échappe à la dégradation et s'accumule y compris dans le noyau.

La caractérisation des voies de signalisation contrôlées par les complexes SCF, chez différents organismes eucaryotes, montre leur rôle central dans le maintien de l'homéostasie cellulaire.

Les Inventeurs ont ainsi identifié chez la levure, la protéine Met30 comme un facteur impliqué dans la répression transcriptionnelle des gènes impliqués dans la biosynthèse des acides aminés soufrés (cystéine, méthionine et S-adénosylméthionine (AdoMet)) (Thomas D. et al., (1995), Mol. Cell. Biol., 15, 6526-6534). Chez la levure, cette voie métabolique compte un ensemble d'environ 25 gènes (gènes *MET)* dont la plupart sont strictement co-régulés, c'est-à-dire qu'en réponse à une augmentation intracellulaire d'AdoMet qui peut-être facilement obtenue par addition de méthionine dans le milieu de croissance des levures, la transcription de ces gènes est bloquée. Chez l'homme, le métabolisme des acides aminés soufrés est très diffèrent du fait que, contrairement à la levure, il n'est pas capable d'assimiler le sulfate et sa croissance requiert un apport alimentaire en acides aminés soufrés (méthionine). Des études antérieures ont mis en évidence l'existence d'au moins cinq facteurs transcriptionnels différents chez la levure, qui sont nécessaires à l'activation transcriptionnelle des gènes *MET ;* parmi ces facteurs, on peut citer, deux protéines à fermeture de leucine (bZIP), Met4 et Met28, deux protéines à doigt de zinc, Met31 et Met32 et la protéine Cbfl, qui est aussi un composant du kinétochore de la levure (Thomas D. et al. (1997), Microbiol. Mol. Biol. Rev., 61, 503-532). Le facteur Met4 en particulier n'a pas d'homologue fonctionnel connu chez l'Homme. Selon les gènes, différentes combinaisons de facteurs s'associent en complexes qui reconnaissent des séquences spécifiques en amont des gènes *MET ;* ainsi le complexe Cbf1-Met4-Met28 se fixe sur la séquence TCACGTC en amont du gène *MET16* alors que les complexes Met4-Met28-Met31 et Met4-Met28-Met32 reconnaissent le motif AAACTGTG en amont des gènes *MET3* et *MET28* (Kuras L. et al. (1997), EMBO J., 16, 2441-2451 ; Blaiseau P. L. et al. (1998), EMBO J., 17,6327-6336). Dans tous ces complexes, l'activation transcriptionnelle des différents gènes *MET* ne dépend que d'un seul domaine d'activation porté par la sous-unité Met4.

Le dysfonctionnement de la voie ubiquitine-protéasome, voie de dégradation des protéines, a été établi dans de nombreuses pathologies de natures extrêmement diverses, notamment les cancers, les maladies génétiques, la maladie de Parkinson, la maladie d'Alzheimer, les syndromes inflammatoires et les infections virales. Ainsi, on sait que toute mutation présente sur les protéines cibles autour des sites de phosphorylation, abolit la reconnaissance des protéines mutées par les complexes SCF et conduit à leur stabilisation. Il a été démontré récemment, que de telles mutations qui affectent la β-caténine et interdisent sa destruction, sont impliquées dans la transformation tumorale dans de nombreux tissus (cancer du colon, mélanome, hépatocarcinome, etc.). A l'opposé, la dégradation excessive de la β-caténine dans les neurones a été mise en cause dans la maladie d'Alzheimer, et est impliquée dans la mort neuronale par apoptose qui survient dans cette pathologie.

Aussi la recherche de molécules, aptes à agir sur les pathologies liées à un dysfonctionnement de la cascade de l'ubiquitine et de la dégradation du protéasome, notamment des anticancéreux, des agents anti-inflammatoires et des agents antiviraux (Maniatis T. (1999) référence citée; F. Margottin et al., (1999), Médecine et Sciences, 15, 1008-1014) s'avère hautement souhaitable.

Différents procédés de criblage de composés actifs sur la cascade de l'ubiquitine et la dégradation du protéasome ont été proposés et mettent en oeuvre, un couple ubiquitine-ligase/substrat spécifique, de préférence d'origine humaine, dans lequel le substrat est une protéine régulatrice d'un processus cellulaire dont la dégradation excessive ou l'absence de dégradation par la voie ubiquitine-protéasome est directement impliquée dans la pathologie à traiter :
- la Demande Internationale PCT WO 97/12962 décrit, pour cribler des agents anticancéreux, un procédé qui met en oeuvre une ubiquitine-ligase E3 contenant une région C-terminal homologue au domaine catalytique de la protéine E6-AP (famille « HECT » : h-pub1, h-pub2, h-pub3, s-pub1) capable d'ubiquitinyler spécifiquement une protéine régulatrice du cycle cellulaire telle que la tyrosine phosphatase cdc 25 ou le suppresseur de tumeur p53,
- la Demande Internationale PCT WO 99/04033 et le brevet américain US n° 5,932,425, pour cribler des substances aptes à traiter des désordres caractérisés par une augmentation de l'activité transcriptionnelle du facteur NF-κB (maladies auto-immunes, états inflammatoires, cachexies, SIDA), décrivent un procédé qui met également en oeuvre une ubiquitine-ligase humaine de la famille HECT (RSC ou KIAAN), capable d'ubiquitinyler spécifiquement la protéine IκB qui forme un complexe cytoplasmique inactif avec le facteur de transcription ubiquitaire NF-κB, qui régule la réponse immunitaire et inflammatoire,
- la Demande Internationale PCT WO 99/38969 décrit un procédé qui met en oeuvre une protéine à boîte F (protéine β-TrCP humaine) qui se lie à la protéine virale Vpu, pour cribler des agents anti -VIH. Vpu qui sert d'intermédiaire dans le ciblage des récepteurs cellulaires CD4 vers la voie de dégradation ubiquitine-protéasome participe ainsi à la diminution du nombre de lymphocytes T CD4+ fonctionnels responsable de l'immunodéficience liée à l'infection par le VIH.

De manière générale, dans ces différents procédés, qui mettent en oeuvre des systèmes d'origine humaine, il existe un lien direct entre la pathologie à traiter et les protéines dont la modulation de la dégradation est recherchée.

Or, poursuivant leurs travaux, les Inventeurs ont montré, que de manière inattendue, le complexe SCF^{Met30} contrôle la transcription de l'ensemble des gènes de la voie d'assimilation du sulfate et que la répression transcriptionnelle de cette voie, en réponse à une augmentation de la concentration intracellulaire en AdoMet, résulte de la dégradation spécifique de Met4 qui fait intervenir le complexe SCF^{Met30}. L'existence de ce contrôle établit pour la première fois le lien direct entre l'activité du complexe SCF^{Met30} et la régulation du métabolisme des acides aminés soufrés chez la levure.

Tirant partie de l'extrême conservation des mécanismes moléculaires impliqués dans la voie ubiquitine-protéasome de dégradation des protéines chez l'ensemble des cellules eucaryotes, les Inventeurs ont mis au point un procédé de criblage cellulaire de composés aptes à agir sur les pathologies liées au dysfonctionnement de la cascade ubiquitine-protéasome chez l'homme en utilisant cette voie d'une manière qui n'avait jamais été envisagée. Ils ont, en effet, sélectionné une protéine de la voie ubiquitine-protéasome, dont la stabilité n'est pas directement impliquée dans les pathologies à traiter, mais qui permet de disposer d'outils de criblage très performants, qui présentent l'avantage d'être rapides et fiables.

La présente invention a en conséquence pour objet un procédé de criblage cellulaire de composés ou d'agents aptes à agir sur les pathologies liées au dysfonctionnement de la cascade ubiquitine-protéasome chez l'Homme, caractérisé en ce qu'il comprend les étapes suivantes :
(i) la mise en contact du produit à tester avec une souche de levure modifiée, comportant (a) une séquence hybride, comprenant une séquence codant pour une protéine Met4, sous sa forme sauvage ou mutée, fusionnée en phase avec au moins une séquence codant pour un marqueur approprié, ladite séquence hybride étant exprimée sous le contrôle d'un promoteur actif chez la levure et éventuellement (b) un système transcriptionnel rapporteur, constitué par un gène rapporteur placé sous le contrôle d'une séquence opératrice appropriée ou d'un promoteur de levure approprié,
(ii) l'addition de méthionine, à des concentrations répressives, comprises entre 0,03 mM et 20 mM, ou non répressives, et
(iii) la détermination du niveau d'expression et de la stabilité de la protéine exprimée à partir de la séquence hybride, soit par visualisation et/ou quantification, soit par détermination de l'activité du gène rapporteur.
   Dans un mode de mise en oeuvre avantageux du procédé selon l'invention, il peut comprendre en parallèle les étapes suivantes :
(iv) la mise en contact du produit à tester avec une souche de levure modifiée, comportant une séquence hybride, comprenant une séquence codant pour une protéine Met30, sous sa forme sauvage ou mutée, fusionnée en phase avec au moins une séquence codant pour un marqueur approprié, ladite séquence hybride étant exprimée sous le contrôle d'un promoteur, actif chez la levure,
(v) l'addition de méthionine, à des concentrations répressives, comprises entre 0,03 mM et 20 mM, ou non répressives, et
(vi) la détermination du niveau d'expression et de la stabilité de la protéine exprimée à partir de la séquence hybride, soit par visualisation et/ou quantification, soit par détermination de l'activité du gène rapporteur.
   Dans un autre mode de mise en oeuvre avantageux de ce procédé selon l'invention, il peut comprendre en outre, un moyen supplémentaire mettant en oeuvre un système cellulaire de contrôle, qui permettra de s'assurer de la spécificité de réponse des systèmes hybrides ou des systèmes rapporteurs transcriptionnels utilisés dans les étapes (i) à (vi), ledit moyen comprenant les étapes suivantes :
(vii) la mise en contact du produit à tester avec une souche de levure modifiée, comportant une séquence hybride, comprenant une séquence codant pour une protéine Met28, sous sa forme sauvage ou mutée, fusionnée en phase avec au moins une séquence codant pour un marqueur approprié, ladite séquence hybride étant exprimée sous le contrôle d'un promoteur, actif chez la levure,
(viii) l'addition de méthionine, à des concentrations répressives, comprises entre 0,03 mM et 20 mM, ou non répressives, et
(ix) la détermination du niveau d'expression et de la stabilité de la protéine exprimée à partir de la séquence hybride, soit par visualisation et/ou quantification, soit par détermination de l'activité du gène rapporteur.
   Dans un autre mode de mise en oeuvre avantageux de ce procédé selon l'invention, il peut comprendre en outre, un moyen supplémentaire mettant en oeuvre un système acellulaire de contrôle qui repose sur la mesure des taux de transcription des séquences hybrides et des gènes métaboliques *MET16* et *MET25,* ledit moyen comprenant les étapes suivantes :
(x) l'extraction des ARNs totaux des souches modifiées utilisées soit à l'étape (i), soit à l'étape (iv), soit à l'étape (vii) et
(xi) la mesure des taux de transcription de la séquence hybride et celle des gènes métaboliques *MET16* et *MET25.*
   Au sens de la présente invention, on entend par forme mutée d'une protéine, une protéine modifiée soit par insertion, délétion ou substitution d'un ou plusieurs acides aminés.
   Au sens de la présente invention, on entend par méthionine, la forme (L) ou la forme (DL) de la méthionine.
   Au sens de la présente invention, on entend par séquence opératrice ou opérateur approprié(e) contrôlant le gène rapporteur, une séquence reconnue par un facteur de liaison à l'ADN fusionné en phase à une protéine Met4, telle que la protéine de fusion LexA-Met4.
   Au sens de la présente invention, on entend par promoteur de levure contrôlant le gène rapporteur, un promoteur activé par le facteur de transcription Met4, tel qu'un gène *MET* comme *MET3, MET10, MET14, MET16, MET25* ou *MET28.*
   Dans un mode de mise en oeuvre avantageux de ce procédé selon l'invention, le marqueur utilisé pour la construction de la séquence hybride est choisi dans le groupe constitué par : les peptides antigéniques, par exemple le motif antigénique hémagglutinine (Ha), les protéines à fluorescence intrinsèque, comme par exemple la protéine à fluorescence verte *(Green Fluorescence Protein* ou GFP), les protéines à activité enzymatique mesurable, et les facteurs de liaison à l'ADN comme par exemple LexA de *E. coli.*
   Dans un autre mode de mise en oeuvre avantageux de ce procédé selon l'invention, le promoteur permettant l'expression de la protéine hybride peut être soit un promoteur inductible actif chez *S. cerevisiae,* qui peut être avantageusement choisi dans le groupe constitué par le promoteur du gène *GAL*1 et le promoteur du gène *CUP*1*,* soit un promoteur constitutif, comme par exemple le promoteur du gène *ADH1* de *S*. *cerevisiae.*
   Dans un autre mode de mise en oeuvre avantageux de ce procédé, le marqueur utilisé pour la construction de ladite séquence hybride est le facteur de liaison à l'ADN LexA, et ledit gène rapporteur est placé sous le contrôle d'opérateurs LexA.
   La présente invention a également pour objet un procédé de criblage cellulaire de composés aptes à agir sur les pathologies liées au dysfonctionnement de la cascade ubiquitine-proteéasome chez l'Homme, caractérisé en ce qu'il comprend les étapes suivantes :
(xii) la mise en contact du produit à tester avec une souche de levure modifiée, comportant un système transcriptionnel rapporteur, constitué par un gène rapporteur placé sous le contrôle d'un promoteur activé par le facteur de transcription Met4,
(xiii) l'addition de méthionine, à des concentrations répressives, comprises entre 0,03 mM et 20 mM, ou non répressives, et
(xiv) la détermination de l'activité du gène rapporteur.

Dans un mode de mise en oeuvre avantageux desdits procédés, le gène rapporteur présent dans le système rapporteur transcriptionnel est choisi dans le groupe constitué par les gènes rapporteurs dont l'activité est visualisable par une méthode colorimétrique, comme par exemple le gène *XylE* de *P. putida* et le gène *LacZ d'E. coli,* et les gènes métaboliques, dont l'activité est mesurable par un test de croissance, notamment les gènes *HIS3, URA3, TRP1 et LEU2* de *S. cerevisiae.*

Lesdits gènes métaboliques peuvent alternativement et avantageusement être utilisés comme gènes de sélection des levures modifiées comportant une séquences hybride et/ou ou un système transcriptionnel rapporteur, tels que définis ci-dessus.

Dans un autre mode de mise en oeuvre avantageux desdits procédés, ledit gène rapporteur (présent dans le système transcriptionnel rapporteur) est placé sous le contrôle du promoteur d'un gène *MET,* de préférence *MET3, MET10, MET16, MET25* ou *MET28.*

Avantageusement, les souches de levures portent une à plusieurs mutations qui augmentent la perméabilité aux produits à tester (Vidal M et al., (1999), Trends in Biotechnol., 17, 374-381).

Pour la mise en oeuvre des procédés selon l'invention, on peut utiliser des souches de levure possédant le fond génétique de la souche W303 de la levure *S. cerevisiae* qui est décrite par Bailis A. M. et al., (Genetics, (1990), 126, 535-547) ou toute autre souche caractérisée de ladite levure. La transformation des cellules de levures par de l'ADN exogène a été réalisée en utilisant des techniques connues de l'homme du métier, notamment la technique décrite par Schiestl R. H. et al. (Curr. Genet., (1989), 16, 339-346), les techniques de génétique (sporulation, dissection et évaluation des marqueurs..) sont également connues et on peut citer notamment celles décrites par Sherman F. et al. (in Methods in Yeast Genetics : a Laboratory Manual, (1979), Cold Spring Harbor, N. Y.) et les techniques de génétique inverse décrites par Rothstein R. ( Methods in Enzymology, (1991), 194, 281-301) ; comme technique d'intégration dirigée au *locus* par simple recombinaison homologue *(crossing over),* on peut citer celle qui est décrite dans Orr-Weaver *et al.* (1983 ; référence citée).

Conformément à l'invention, les levures peuvent être transformées par des plasmides construits par des techniques de biologie moléculaire classiques, notamment selon les protocoles décrits par Sambrook J. et al. (Molecular cloning Laboratory Manual, 2nd edition, (1989), Cold Spring Harbor, N. Y.) et Ausubel F. M. et al. (Current Protocols in Molecular Biology, (1990-1999), John Wiley and Sons, Inc. New York).

Conformément à l'invention, l'activité des gènes rapporteurs et des gènes *MET* est mesurée, selon le gène rapporteur et le promoteur utilisés, par des techniques connues en soi, notamment des techniques colorimétriques, enzymatiques, immunologiques, de fluorescence ou de sélection sur un milieu de croissance approprié.

Conformément à l'invention, l'activité du complexe SCF^{Met30} est déterminée par la mesure du niveau d'expression et de la stabilité de la protéine codée par la séquence hybride et/ou par l'activité du système rapporteur transcriptionnel ; en effet, la protéine hybride codée par ladite séquence hybride conserve avantageusement les propriétés intrinsèques de chacun des deux éléments fusionnés la constituant. Par exemple, la protéine Met4-marqueur est dégradée sélectivement (par addition de méthionine dans le milieu de culture) par la voie ubiquitine-protéasome (propriété de la protéine Met4) et est visualisable, conformément au marqueur associé : lorsque le marqueur présent dans la protéine hybride est la protéine GFP, alors l'activité du complexe SCF^{Met30} est visualisée en observant et en quantifiant la fluorescence de la protéine hybride GFP-Met4 ou GFP-Met30 ; lorsque le marqueur présent dans la protéine hybride, est le motif antigénique hémagglutinine (Ha), alors l'activité du complexe SCF^{Met30} est visualisée par des techniques immunologiques, telles que des techniques de transfert de protéines *(Western Blotting),* des techniques ELISA ou des techniques d'immunoprécipitation (Harlow E. et al., (1988), Antibodies, a Laboratory Manual, Cold Spring Harbor, N. Y.) ; lorsque les cellules contiennent un système rapporteur transcriptionnel contenant le gène *XylE,* alors l'activité du complexe SCF^{Met30} est visualisée par vaporisation, sur les cellules de levure, de catéchol à une concentration comprise entre 50 mM et 1 M, et en mesurant l'apparition d'une coloration jaune (Worsay M. J. et al., (1975), J. Bacteriol., 124, 7-13) ; lorsque les cellules contiennent un système rapporteur transcriptionnel contenant le gène *LacZ d'E. coli,* alors l'activité du complexe SCF^{Met30} est visualisée en mesurant l'apparition d'une coloration bleue, sur un milieu contenant le substrat colorigénique X-Gal (Sambrook J. (1989), référence citée) ; lorsque les cellules contiennent un système rapporteur transcriptionnel contenant le gène *HIS3* de S. *cerevisiae,* alors l'activité du complexe SCF^{Met30} est visualisée en faisant croître la levure sur un milieu minimum ne contenant pas d'histidine, en présence d'aminotriazole, à des concentrations de 0,5 mM à 200 mM.

Du fait de l'existence de nombreuses sous-unités communes aux différents complexes SCF, lesdites sous-unités étant en équilibre stoechiométrique, le complexe SCF^{Met30} peut servir de modèle pour cribler des molécules pour leur capacité à moduler soit l'activité des voies de signalisation contrôlées par l'ensemble des complexes SCF, soit celle de la voie ubiquitine-protéasome, voie de dégradation des protéines.

De manière surprenante, ce deuxième système (voie ubiquitine-protéasome), tel que mis en oeuvre dans la présente invention, en utilisant comme substrat, la protéine Met4, est particulièrement avantageux pour cribler des molécules ou agents aptes à agir sur les pathologies liées au dysfonctionnement de la cascade ubiquitine-protéasome chez l'homme telles que les cancers, les maladies génétiques, la maladie de Parkinson, la maladie d'Alzheimer, les syndromes inflammatoires et les infections virales :
- simplicité : l'induction du système complexe SCF^{Met30} /Met4 est réalisée simplement par addition de méthionine dans le milieu de croissance, et lorsque le marqueur est la protéine GFP, l'activité du complexe SCF^{Met30} est visualisée directement par l'observation et ou la quantification de la fluorescence émise,
- rapidité de développement : les techniques de génétique inverse, la connaissance intégrale du génome de la levure et les méthodes de biologie moléculaires adaptées à cet organisme, assurent une mise en oeuvre rapide de souches stables indicatrices,
- rapidité de croissance et de criblage : la levure est un microorganisme à croissance rapide et rendement élevé, ce qui autorise l'obtention de cellules modifiées pour un nombre élevé de criblages,
- faible coût : la levure est un microorganisme dont la culture, le stockage, la caractérisation sont peu onéreux.

Les procédés de criblage selon l'invention, peuvent notamment servir à sélectionner des agents actifs comme anticancéreux, anti-inflammatoires, antiviraux ou des agents actifs dans des maladies génétiques, notamment dans la maladie de Parkinson et la maladie d'Alzheimer.

Au sens de la présente invention on entend par composé ou agent, toute molécule issue de procédés de synthèses ou de ressources naturelles.

Les inventeurs décrivent également l'utilisation d'agents sélectionnés par les procédés selon la présente invention, pour la préparation de médicaments destinés au traitement des maladies liées à des troubles de l'activité des complexes SCF ou de la voie ubiquitine-protéasome, telles que les cancers, les maladies génétiques, la maladie de Parkinson, la maladie d'Alzheimer, les syndromes inflammatoires et les infections virales.

Il est également décrit des plasmides, caractérisés en ce qu'ils contiennent une séquence hybride, comprenant une séquence codant pour une protéine Met4, Met28 ou Met30 sous sa forme sauvage ou mutée, fusionnée en phase avec au moins une séquence codant pour un marqueur choisi selon la protéine Met, dans le groupe constitué par : les peptides antigéniques, les protéines à fluorescence intrinsèque et les protéines à activité enzymatique mesurable, ladite séquence hybride pouvant être exprimée chez la levure, sous le contrôle d'un promoteur, constitutif ou inductible.

Conformément à la description :
- lorsque ledit plasmide comprend une séquence codant pour une protéine Met4, alors ledit marqueur est choisi parmi les peptides antigéniques, les protéines à fluorescence intrinsèque,
- lorsque ledit plasmide comprend une séquence codant pour une protéine Met28, alors ledit marqueur est choisi parmi les peptides antigéniques, les protéines à fluorescence intrinsèque et les protéines à activité enzymatique mesurable, et
- lorsque ledit plasmide comprend une séquence codant pour une protéine Met30, alors ledit marqueur est choisi parmi les protéines à fluorescence intrinsèque et les protéines à activité enzymatique mesurable.

Lesdits plasmides sont sélectionnés dans le groupe constitué par :
- les plasmides contenant une séquence codant pour une protéine Met4 ou Met28 fusionnée à un peptide comprenant 3 motifs antigéniques de l'hémagglutinine (HA), ladite séquence étant exprimée sous le contrôle du promoteur inductible *GAL1,*
- les plasmides contenant une séquence codant pour une protéine Met4, Met28 ou Met30, fusionnée à une protéine GFP, ladite séquence étant exprimée respectivement sous le contrôle des promoteurs constitutifs *MET4, MET28* ou *MET30*
ou sous le contrôle du promoteur inductible *GAL1,*
- les plasmides contenant une séquence codant pour une protéine Met30, fusionnée à une protéine GFP et à un peptide comprenant 3 motifs antigéniques de l'hémagglutinine (HA), ladite séquence étant exprimée sous le contrôle du promoteur *GAL1.*

Il est également décrit des plasmides, caractérisés en ce qu'ils contiennent une séquence hybride, comprenant une séquence codant pour une protéine Met4, sous sa forme sauvage ou mutée, fusionnée en phase avec au moins une séquence codant pour le facteur de liaison à l'ADN LexA, et le gène *TRP1* ou le gène *LEU2* comme gènes de sélection des levures modifiées par lesdits plasmides,

Il est également décrit des plasmides contenant au moins un système transcriptionnel rapporteur, constitué par un gène rapporteur placé sous le contrôle, soit d'un promoteur de levure approprié, soit d'une séquence opératrice appropriée, tels que définis précédemment.

De manière avantageuse, lesdits plasmides contiennent le gène rapporteur *LacZ* ou *XylE,* exprimé sous le contrôle, soit du promoteur *MET16,* soit d'opérateurs LexA.

Il est également décrit des souches de levure, caractérisées en ce qu'elles sont modifiées, de façon stable, par un plasmide tel que décrit ci-dessus.

D'autres caractéristiques et avantages de l'invention apparaissent dans la suite de la description et des exemples, illustrés par les figures dans lesquelles :

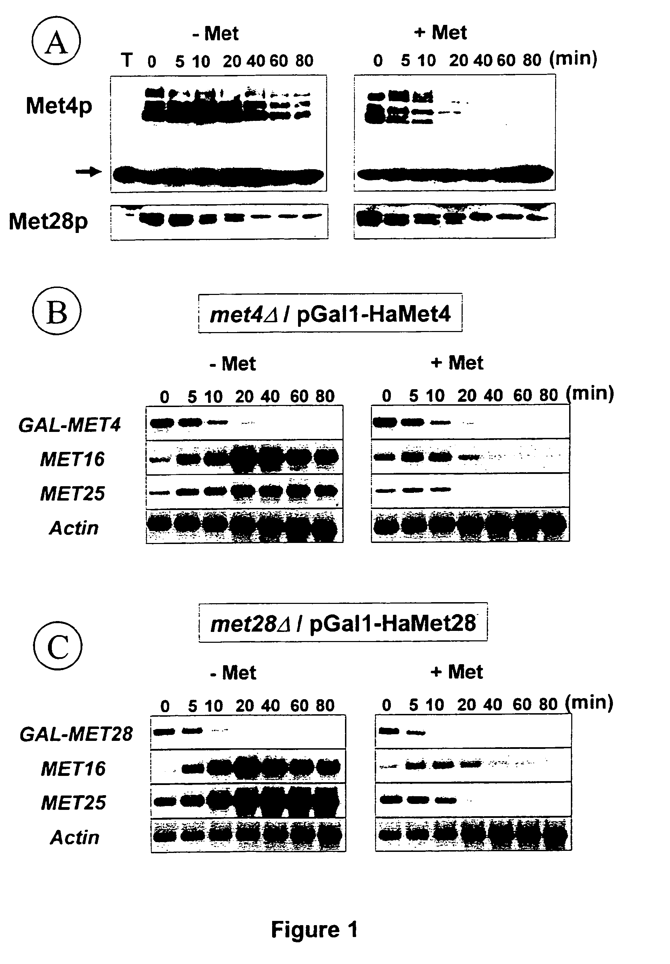

- la figure 1 illustre l'influence de l'addition de méthionine à des concentrations répressives dans le milieu de culture (A) les cellules comportant un plasmide codant pour les protéines marquées par des motifs antigéniques hémagglutinine (Ha) sous le contrôle du promoteur *GAL*1*,* et préparé selon le mode opératoire décrit dans les exemples 2 et 4, sont cultivées, selon le mode opératoire décrit dans l'exemple 12, dans un milieu minimum contenant 2 % de galactose pendant 90 minutes, soit en présence de méthionine à une concentration répressive (+ Met), soit en l'absence de méthionine (- Met) ; (B) et (C) les ARN totaux sont extraits, selon le mode opératoire décrit dans l'exemple 12 à partir de cellules utilisées en (A) et exprimant soit la protéine hybride Ha-Met4, soit la protéine hybride Ha-Met28. Les cellules sont cultivées dans les conditions décrites en (A) et sont analysées avec *MET4, MET16, MET25, MET28.* « met4Δ» correspond à une cellule W303 modifiée par une copie chromosomique du gène *MET4* inactivé ; «met28Δ » correspond à une cellule W303 modifiée par une copie chromosomique du gène *MET28* inactivé.

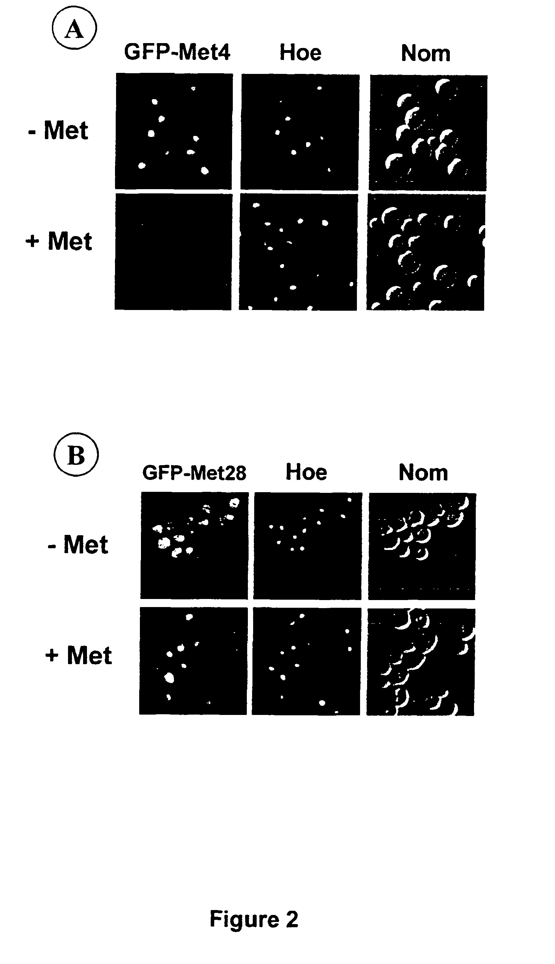

- la figure 2 illustre la localisation des protéines hybrides GFP-Met4 et GFP-Met28 dans des cellules sauvages, en l'absence de méthionine (- Met) ou en présence de méthionine à une concentration répressive (+ Met). Les cellules sont cultivées dans les conditions décrites dans l'exemple 12. « Hoe » correspond à l'indicateur coloré spécifique des noyaux, Hoechst 333-42 ; « Nom » correspond à l'image obtenue par microscopie interférentielle selon Nomarski.

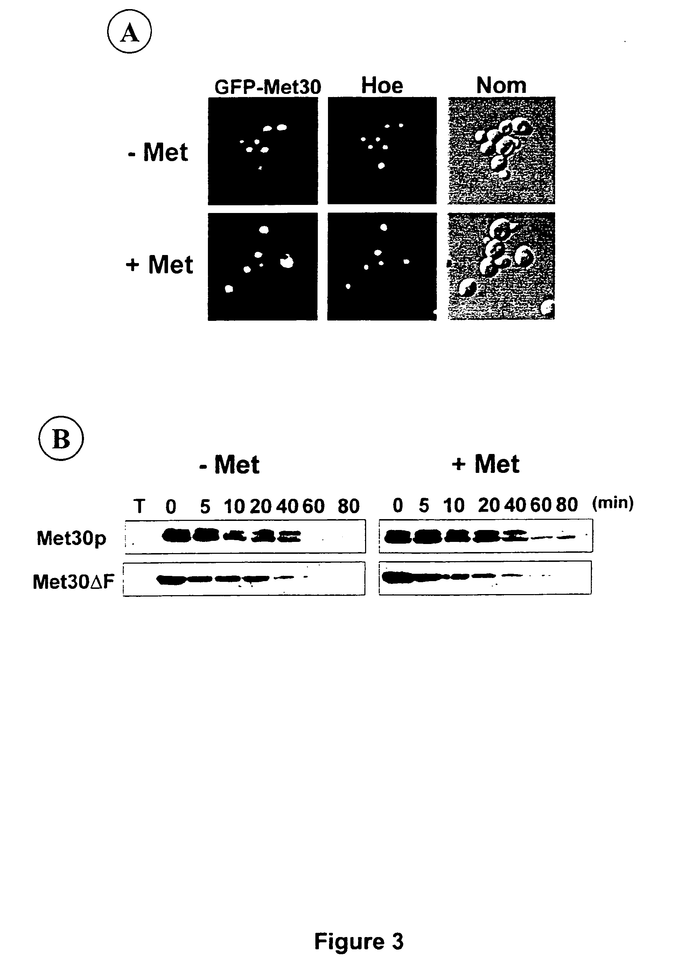

- la figure 3 illustre (A) la localisation de la protéine hybride GFP-Met30 dans des cellules sauvages, en l'absence de méthionine (- Met) ou en présence de méthionine à une concentration répressive (+ Met). Les cellules sont cultivées dans les conditions décrites dans l'exemple 12. « Hoe » correspond à l'indicateur coloré Hoechst 333-42 ; « Nom » correspond à l'image obtenue par microscopie interférentielle selon Nomarski ; (B) stabilité des protéines hybrides Ha-Met30 et Ha-Met30ΔF dans des souches W303-1A sauvages.

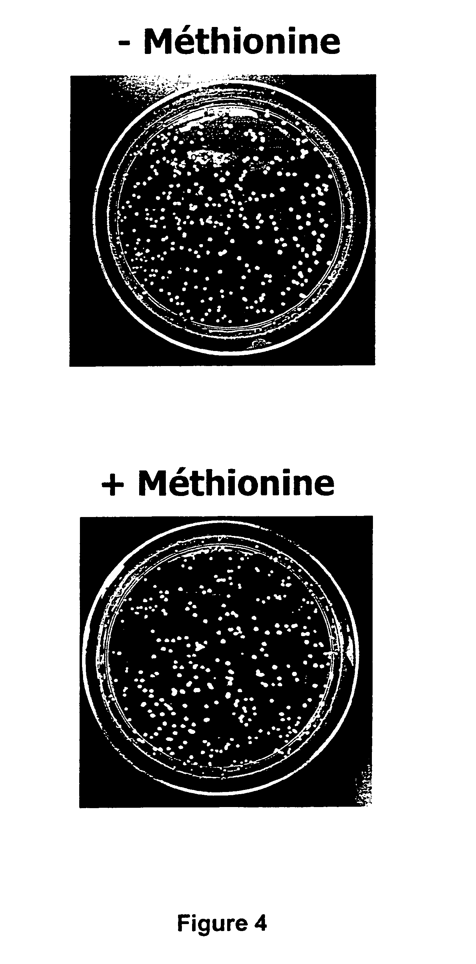

- la figure 4 illustre l'activité du gène rapporteur LexAopXylE en l'absence de méthionine (- Met) ou en présence de méthionine à une concentration répressive, à partir d'une souche de levure (C190) exprimant la protéine hybride codée par le plamide pLexMet4-7 ; la visualisation est réalisée par la mesure de la catéchol-oxydase.

### Exemple 1 : Construction des plasmides de base pGal316Flu, pGa1306Flu et pFL39Flu.

### 1.1. Construction des plasmides pGal316Flu et pGa1306Flu

### 1.1.1. Construction des plasmides pGal316 et pGal306 :

Un fragment de 900 paires de bases (bp) du plasmide pJN1 (Nehlin J. et al., (1990) EMBO J., 9, 2891-2898) contenant une fusion entre les promoteurs des gènes de S. cerevisiae, GAL1 et TPK2, est amplifié par PCR (polymerase chain reaction), en utilisant les oligonucléotides «olGal1O» de séquence SEQ ID N°1: 5' CAAAGAAGCTTAATAATCATATT 3' et « olGaITPK» de séquence SEQ ID N° 2:5'TTGACCAACTGGCTGAGCC 3'.

Le fragment obtenu est digéré par les enzymes de restrictions HindIII et EcoRI et inséré :
(i) dans le plasmide navette S . cerevisiae - E. coli, pRS316 dont la séquence est déposée à la banque de données EMBL, avec l'identificateur « PRS316 », et le n° U03442, préalablement digéré par les enzymes HindIII et EcoRI, produisant le plasmide pGa1316,
(ii) dans le plasmide navette S . cerevisiae - E. coli, pRS306 dont la séquence a été déposée à la banque de données EMBL, avec l'identificateur « PRS306 », sous le n° U03438, préalablement digéré par les enzymes de restriction HindIII et EcoRI.

On produit ainsi le plasmide pGa1306 .

### 1.1.2. Construction des plasmides pGa1316F1u et pGa1306F1u :

Un fragment d'ADN double brin correspondant à la séquence SEQ ID N° 3 et codant pour un répétition de 3 motifs antigéniques hémagglutinine (Ha) est inséré :
(i) dans le plasmide pGal316 obtenu au point 1.1.1, préalablement digéré par l'enzyme de restriction *Eco*RI et dont les extrémités ont été rendues franches par un traitement avec le fragment de Klenow de l'ADN polymérase d*'E. coli,* produisant le plasmide pGa1316Flu,
(ii) dans le plasmide pGa1306 préparé au moint 1.1.1., préalablement digéré par l'enzyme de restriction *EcoRI* et dont les extrémités ont été rendues franches par un traitement avec le fragment de Klenow de l'ADN polymérase d*'E. coli,* produisant le plasmide pGa1306Flu.

### 1.2. Construction du plasmide pFL39Flu :

Dans une première étape le site *EcoRI* présent dans le polylinker du plasmide navette *S . cerevisiae - E. coli* pFL39 a été détruit. Pour cela le plasmide pFL39, dont la séquence est celle déposée à la banque de données EMBL, avec l'identificateur « CVPFL39 », sous le n° X70483, a été digéré par *EcoRI,* les extrémités rendues franches par un traitement avec le fragment de Klenow de l'ADN polymérase d'*E*. *coli,* et le produit ainsi traité a été religaturé sur lui-même produisant le plasmide pFL39E0.

Dans une seconde étape, le fragment *Hin*dIII*-Pst*I du plasmide pGal316Flu préparé selon le mode opératoire décrit au point 1.1. et comprenant la région promoteur *GAL1* et le fragment codant pour la région antigénique Ha, a été inséré dans le plasmide pFL39E0 préalablement digéré par les enzymes *Hind*III et *Pst*I*.*

On obtient ainsi le plasmide pFL39Flu.

### Exemple 2: Plasmides permettant l'expression chez la levure de protéines hybrides Ha-Met4 sous le contrôle du promoteur GAL1.

Les plasmides qui suivent permettent l'expression chez la levure S. *cerevisiae* de dérivés de la protéine Met4 comprenant une répétition de trois motifs antigénique hémagglutinine (Ha) à leur extrémité amino terminale.

### 2.1. Construction du plasmide pGa1316FluMet4 :

Le fragment d'ADN *Eco*RI*-Bam*HI du vecteur pLexM4-1 (Thomas D. et al., (1992), Mol. Cell. Biol., 12, 1719-1727) codant pour les acides aminés 15 à 666 de la protéine Met4 a été cloné dans le plasmide pGa1316Flu préalablement digéré par les enzymes *Eco*RI et *Bam*HI*,* produisant le plasmide pGal316FluMet4. Ce plasmide peut se répliquer de façon autonome chez la levure.

### 2.2. Construction du plasmide pGal316FluMet4Δ12:

Le fragment d'ADN *Eco*RI*-Xba*I du vecteur pLexM4Δ12 (Kuras L. et al., (1995), Mol. Cell. Biol., 15, 208-216) codant pour un dérivé comprenant les acides aminés 15 à 79 et 180 à 666 de la protéine Met4 a été cloné dans le plasmide pGal316Flu préalablement digéré par les enzymes *EcoR*I et *Xba*I*,* produisant le plasmide pGal316FluMet4Δ12, Ce plasmide peut se répliquer de façon autonome chez la levure.

### 2.3. Construction du plasmide pGa1316F1uMet4Δ30 :

Le fragment d'ADN *EcoRI-XbaI* du vecteur pLexM4Δ30 (Kuras L. *et al.,* (1995) référence citée) codant pour un dérivé comprenant les acides aminés 15 à 211 et 232 à 666 de la protéine Met4 a été cloné dans le plasmide pGa1316F1u préalablement digéré par les enzymes *Eco*RI et *Xba*I*,* produisant le plasmide pGa13 16FluMet4A30. Ce plasmide peut se répliquer de façon autonome chez la levure.

### 2.4. Construction du plasmide pGal316FluMet4Δ37:

Le fragment d'ADN *Eco*RI*-Xba*I du vecteur pLexM4Δ37 (Kuras L. *et al.,* (1995), référence citée) codant pour un dérivé comprenant les acides aminés 15 à 344 et 366 à 666 de la protéine Met4 a été cloné dans le plasmide pGal316Flu préalablement digéré par les enzymes *EcoRI* et *Xba*I*,* produisant le plasmide pGal316FluMet4Δ37. Ce plasmide peut se répliquer de façon autonome chez la levure.

### 2.5. Construction du plasmide pGal316FluMet4ΔLZ :

Le fragment d'ADN EcoRI-XbaI du vecteur pLexM4-3 (Thomas D. *et al.,* (1992), référence citée) codant pour les acides aminés 15 à 616 de la protéine Met4 a été cloné dans le plasmide pGa1316Flu préalablement digéré par les enzymes *EcoRI* et *XbaI,* produisant le plasmide pGa1316FluMet4ΔLZ. Ce plasmide peut se répliquer de façon autonome chez la levure.

### 2.6. Construction du plasmide pFL39FluMet4 :

Le fragment d'ADN *EcoRI-BamHI* du vecteur pLexM4-1 (Thomas D. *et al.,* (1992), référence citée) codant pour les acides aminés 15 à 666 de la protéine Met4 a été cloné dans le plasmide pFL39Flu préalablement digéré par les enzymes *EcoRI* et *BamHI,* produisant le plasmide pFL39FluMet4. Ce plasmide peut se répliquer de façon autonome chez la levure.

### Exemple 3 : Construction des plasmides permettant l'expression chez la levure de protéines hybrides GFP-Met4.

Les plasmides qui suivent permettent l'expression chez la levure S. cerevisiae de dérivés de la protéine Met4 fusionnés à la protéine à fluorescence verte d'Aequora victoria (Green Fluorescent Protein ou GFP).

### 3.1. Construction du plasmide pGFPMet4

Un fragment de 710 paires de bases (bp) du plasmide pGFPmut3, codant pour la protéine GFP3 *(Green Fluorescent Protein* mut3, produit du gène *GFP d'Aequora victoria* dont la séquence est déposée à la banque EMBL, avec l'identificateur «AVU73901 », sous le n° U73901) a été amplifié par PCR *(polymerase chain reaction)* en utilisant les oligonucléotides « olGFPM4-5» de séquence SEQ ID N° 4: 5' ACGCGAATTCATGTCTAAAGGTGAATTA 3' et « olGFPM4-3 » de séquence SEQ ID N° 5 5' ACGCGAATTCTTTGTACAATTCATCCAT 3'.

Le fragment obtenu a été digéré par l'enzyme de restriction *Eco*RI et inséré dans le plasmide pM4-5 (Kuras L*. et al.,* (1995) référence citée) préalablement digéré par l'enzyme *Eco*RI, produisant le plasmide pGFPMet4.

Ce plasmide permet l'expression sous le contrôle du promoteur *MET4* d'une protéine hybride GFP-Met4 comprenant les acides aminés 15 à 666 de Met4, et peut se répliquer de façon autonome chez la levure.

### 3.2. Construction du plasmide pGa1316GFPMet4

Le fragment *EcoRI-Bam*HI du plasmide pGFPMet4, codant pour la fusion GFP-Met4, a été inséré dans le plasmide pGa1316 préalablement digéré par les enzymes *Eco*RI et *Bam*HI, produisant le plasmide pGa1316GFPMet4. Ce plasmide peut se répliquer de façon autonome chez la levure.

### 3.3. Construction du plasmide pGa1316GFPMet4Δ12 :

Le fragment d'ADN *EcoRI-EcoRI* du vecteur pGa1316GFPMet4 préparé selon le mode opératoire décrit précédemment et codant pour la GFP a été cloné en phase dans le plasmide pGa1316FluMet412 préalablement digéré par l'enzyme *EcoRI,* produisant le plasmide pGa1316GFPMet4Δ12. Ce plasmide peut se répliquer de façon autonome chez la levure.

### 3.4. Construction du plasmide pGal316GFPMet4Δ30:

Le fragment d'ADN *Eco*RI*-Eco*RI du vecteur pGa1316GFPMet4 préparé selon le mode opératoire décrit au point 3.2 et codant pour la GFP a été cloné en phase dans le plasmide pGal316F1uMet4Δ30 préalablement digéré par l'enzyme *Eco*RI*,* produisant le plasmide pGal316GFPMet4Δ30. Ce plasmide peut se répliquer de façon autonome chez la levure.

### 3.5. Construction du plasmide pGal316GFPMet4Δ37 :

Le fragment d'ADN *EcoRI-EcoRI* du vecteur pGa1316GFPMet4 préparé selon le mode opératoire décrit au point 3.2 et codant pour la GFP a été cloné en phase dans le plasmide pGa1316FluMet4Δ37 préalablement digéré par l'enzyme *EcoRI,* produisant le plasmide pGa1316GFPMet4Δ37. Ce plasmide peut se répliquer de façon autonome chez la levure.

### 3.6. Construction du plasmide pGal306GFPMet4 :

Le fragment d'ADN Notl-Asp718 du vecteur pGal316GFPMet4 préparé selon le mode opératoire décrit au point 3.2 et comprenant l'ensemble du promoteur GAL1 compris dans la séquence déposée à la banque de données EMBL, sous l'identificateur « SCGAL10 », sous le n° K02115 et de la fusion GFP-Met4 (résidus 15 à 666 de Met4), a été inséré dans le plasmide pRS306 préalablement digéré par les enzymes Notl-Asp718, produisant le plasmide pGa1306GFPMet4.

### 3.7. Construction du plasmide pGal306GFPMet4Δ12 :

Le fragment d'ADN *Not*1 *-Asp*718 du vecteur pGa1316GFPMet4Δ12 préparé selon le mode opératoire décrit au point 3.3 et comprenant l'ensemble du promoteur *GAL1* et de la fusion GFP-Met4Δ12 (résidus 1579 et 180-666 de Met4), a été inséré dans le plasmide pRS306 préalablement digéré par les enzymes *Not*l*-Asp*718*,* produisant le plasmide pGal306GFPMet4Δ12.

### 3.8. Construction du plasmide pGa1306GFPMet4Δ30 :

Le fragment d'ADN *Notl-Asp718* du vecteur pGal316GFPMet4Δ30 préparé selon le mode opératoire décrit au point 3.4 et comprenant l'ensemble du promoteur *GAL1* et de la fusion GFP-Met4Δ30 (résidus 15 à 211 et 221 à 666 de Met4), a été inséré dans le plasmide pRS306 préalablement digéré par les enzymes *Not* 1*-Asp*718*,* produisant le plasmide pGal306GFPMet4Δ30.

### 3.9. Construction du plasmide pGa1306GFPMet4Δ37:

Le fragment d'ADN *Not*1*-Asp*718 du vecteur pGa1316GFPMet4Δ37 préparé selon le mode opératoire décrit au point 3.5 et comprenant l'ensemble du promoteur *GAL1* et de la fusion GFP-Met4Δ37 (résidus 15 à 352 et 366 à 666 de Met4), a été inséré dans le plasmide pRS306 préalablement digéré par les enzymes *Not*1*-Asp*718, produisant le plasmide pGa1306GFPMet4037.

### Exemple 4 : Construction des plasmides permettant l'expression chez la levure de protéines hybrides Ha-Met28 et GFP-Met28.

### 4.1. Construction du plasmide pGa1316F1uMet28

Le fragment d'ADN *Eco*RI*-Bam*HI du vecteur pLexM28-2 (Kuras L. et al., (1996), EMBO J., 15, 2519-2529) codant pour les acides aminés 1 à 166 de la protéine Met28 a été cloné dans le plasmide pGal316Flu préalablement digéré par les enzymes *Eco*RI et *Bam*HI, produisant le plasmide pGa1316F1uMet28. Ce plasmide peut se répliquer de façon autonome chez la levure. Il permet l'expression chez la levure d'une protéine Met28 entière comprenant à son extrémité amino-terminale une répétition de 3 motifs antigéniques hémagglutinine (Ha). La protéine hybride Ha-Met28 est exprimée sous le contrôle du promoteur *GAL1.*

### 4.2. Construction du plasmide pFL39FluMet28 :

Le fragment d'ADN EcoRI-BgIII du vecteur pLexM28-2 (Kuras L. *et al*., (1996), référence citée) codant pour les acides aminés 1 à 166 de la protéine Met28 a été cloné dans le plasmide pFL39Flu préalablement digéré par les enzymes *EcoRI* et BgIII, produisant le plasmide pFL39FluMet28. Ce plasmide peut se répliquer de façon autonome chez la levure. Il permet l'expression chez la levure d'une protéine Met28 entière comprenant à son extrémité amino-terminale une répétition de 3 motifs antigéniques hémagglutinine (Ha). La protéine hybride Ha-Met28 est exprimée sous le contrôle du promoteur *GAL1.*

### 4.3. Construction du plasmide pGFPMet28 :

### 4.3.1. construction du plasmide p314Met28 :

Le fragment d'ADN *Xba*I*-Hpa*I du vecteur pMet28-1 (Kuras L. *et al.,* (1996), référence citée) contenant le gène *MET28,* dont la séquence est celle déposée à la banque de données EMBL, sous l'identificateur « SC17015 », sous le n° U17015 et sa région promoteur a été isolé, ses extrémités ont été rendues franches par un traitement avec le fragment de Klenow de l'ADN polymérase *d'E. coli* et ce fragment d'ADN a été inséré dans le plasmide pRS314 (Sikorski R. S. et al., (1989), Genetics, 122, 19-27) préalablement digéré par l'enzyme *Sma*I*,* produisant le plasmide p314Met28. Ce plasmide peut se répliquer de façon autonome chez la levure.

### 4.3.2. construction du plasmide p314GFPMet28 :

Un fragment de 710 paires de bases (bp) du plasmide pGFPmut3, codant pour la protéine GFP3 a été amplifié par PCR en utilisant les oligonucléotides « oIM28GFP5» de séquence SEQ ID N° 6 : et « olM28GFP3 » de séquence SEQ ID N° 7 :

Le fragment obtenu a été inséré dans le plasmide p314Met28 par la méthode du *« gap repair »* selon la technique décrite par Orr-Weaver et al., (Methods in Enzymology, (1983), 101, 228-245).

Ainsi le fragment obtenu après PCR et le plasmide p314Met28, préalablement digéré par l'enzyme *Bgl*II ont été cotransformés dans la souche de levure W303-1A (Bailis A. *M. et al.,* (1990), référence citée), et les clones prototrophes pour le tryptophane ont été sélectionnés. L'ADN plasmidique contenu dans ces clones a été extrait, transformé dans *E. coli* et le plasmide recombinant p314Met28GFP identifié et caractérisé par digestions enzymatiques et séquence. Ce plasmide permet l'expression d'une protéine Met28 entière fusionnée à son extrémité carboxy-terminale avec la protéine GFP. La protéine hybride Met28-GFP est exprimée sous le contrôle du promoteur *MET28.* Ce plasmide peut se répliquer de façon autonome chez la levure.

### Exemple 5 : Construction des plasmides permettant l'expression chez la levure de protéines hybrides Ha-Met30 et GFP-Met30.

### 5.1. Construction du plasmide pGa1316FluMet30ΔN :

Le fragment d'ADN EcoRI-*Bgl*II du vecteur pGadMet30-1 (Thomas *D. et al.,* (1995), référence citée) codant pour les acides aminés 158 à 640 de la protéine Met30 a été cloné dans le plasmide pGal316Flu préalablement digéré par les enzymes *Eco*RI et *Bam*HI, produisant le plasmide pGal316FluMet30ΔN. Ce plasmide peut se répliquer de façon autonome chez la levure. Il permet l'expression chez la levure d'une protéine Met30 tronquée de.sa partie amino-terminale mais comprenant à cette extrémité une répétition de 3 motifs antigéniques hémagglutinine (Ha). La protéine hybride Ha-Met30 est exprimée sous le contrôle du promoteur *GAL1.*

5.2. Construction du plasmide pGa1316FluMet30:Le fragment d'ADN *Eco*RI-EcoRI du vecteur pLexMet30-4 (Thomas D. *et al.,* (1995), référence citée) codant pour les 157 premiers acides aminés de la protéine Met30 a été cloné dans le plasmide pGal316FluMet30ΔN préalablement digéré par l'enzyme *Eco*RI, produisant le plasmide pGal316F1uMet30. Ce plasmide peut se répliquer de façon autonome chez la levure. Il permet l'expression chez la levure d'une protéine Met30 entière comprenant à son extrémité amino-terminale une répétition de 3 motifs antigéniques hémagglutinine (Ha). La protéine hybride Ha-Met30 est exprimée sous le contrôle du promoteur *GAL*1*.*

### 5.3. Construction du plasmide pFL39F1uMet30ΔN :

Le fragment d'ADN *Eco*RI*-Bgl*II du vecteur pGadMet30-1 (Thomas D*. et al.,* (1995), référence citée) codant pour les acides aminés 158 à 640 de la protéine Met30 a été cloné dans le plasmide pFL39Flu préalablement digéré par les enzymes *EcoRI* et *Bam*HI, produisant le plasmide pFL39FluMet30ΔN, Ce plasmide peut se répliquer de façon autonome chez la levure. Il permet l'expression chez la levure d'une protéine d'une protéine Met30 tronquée de sa partie amino-terminale mais comprenant à cette extrémité une répétition de 3 motifs antigéniques hémagglutinine (Ha). La protéine hybride Ha-Met30 est exprimée sous le contrôle du promoteur *GAL1.*

### 5.4. Construction du plasmide pFL39FluMet30ΔF :

Ce plasmide est construit selon le mode opératoire décrit au point 5.3, mais exprime une protéine Met30 mutée qui ne comprend pas les acides aminés 187 à 201.

### 5.5. Construction du plasmide pFL39FluMet30

Le fragment d'ADN *EcoRI-EcoRI* du vecteur pLexMet30-4 (Thomas *D. et al.,* (1995), référence citée) codant pour les 157 premiers acides aminés de la protéine Met30 a été cloné dans le plasmide pFL39FluMet30ΔN préalablement digéré par l'enzyme *EcoRI,* produisant le plasmide pFL39FluMet30. Ce plasmide peut se répliquer de façon autonome chez la levure. Il permet l'expression chez la levure d'une protéine Met30 entière comprenant à son extrémité amino-terminale une répétition de 3 motifs antigéniques hémagglutinine (Ha). La protéine hybride Ha-Met30 est exprimée sous le contrôle du promoteur *GAL1.*

### 5.6. Construction du plasmide p316FluMet30GFP :

Un fragment de 710 paires de bases (bp) du plasmide pGFPmut3 codant pour la protéine GFP3 a été amplifié par PCR en utilisant les oligonucléotides « olM30GFP5» de SEQ ID N° 8 : et « ol316GFP3 » de SEQ ID N° 9

Le fragment obtenu a été inséré dans le plasmide pGal316FluMet30 par la méthode du *« gap repair* » (Orr Weaver *et al.,* 1983 ; référence déjà citée).

Ainsi le fragment obtenu après PCR et le plasmide pGa1316FluMet30, préalablement digéré par l'enzyme Xba*I* ont été cotransformés dans la souche W303-1A de levure et les clones prototrophes pour l'uracile ont été sélectionnés. L'ADN plasmidique contenu dans ces clones a été extrait, transformé dans *E. coli* et le plasmide recombinant p316F1uMet30GFP identifié et caractérisé par digestions enzymatiques et séquence. Ce plasmide permet l'expression d'une protéine Met30 entière, fusionnée à son extrémité carboxy-terminale, avec la protéine GFP et à son extrémité amino-terminale avec une répétition de 3 motifs antigéniques hémagglutinine (Ha). La protéine hybride Ha-Met30-GFP est exprimée sous le contrôle du promoteur *GAL*1*.* Ce plasmide peut se répliquer de façon autonome chez la levure.

### 5.7. Construction du plasmide pMet30GFP :

### 5.7.1. construction du plasmide pMet30-8 :

Le fragment d'ADN *Xba*I*-Sal*I du vecteur pMet30-1 (Thomas D. *et al.,* (1995), référence citée) contenant le gène *MET30* dont la séquence est celle déposée à la banque de données EMBL, sous l'identificateur « SCMET30A », sous le n° L26505 et sa région promoteur a été isolé et inséré dans le plasmide pRS316 préalablement digéré par les enzymes *XbaI* et SalI, produisant le plasmide pMet30-8. Ce plasmide peut se répliquer de façon autonome chez la levure.

### 5.7.2. construction du plasmide pMet30GFP :

Un fragment de 710 paires de bases (bp) du plasmide pGFPmut3 codant pour la protéine GFP3 a été amplifié par PCR en utilisant les oligonucléotides « olM30GFP5» de séquence SEQ ID N° 10 : et « olM30GFP3 » de séquence SEQ ID N° 11:

Le fragment obtenu a été inséré dans le plasmide pMet30-8 par la méthode du *« gap repair* » (Orr Weaver *et al.,* 1983, référence déjà citée).

Ainsi, le fragment obtenu après PCR et le plasmide pMet30-8, préalablement digéré par l'enzyme *Nhe*I ont été cotransformés dans la souche W303-1A de levure et les clones prototrophes pour l'uracile ont été sélectionnés.

L'ADN plasmidique contenu dans ces clones a été extrait, transformé dans *E*. *coli* et le plasmide recombinant pMet30GFP identifié et caractérisé par digestions enzymatiques et séquence. Ce plasmide permet l'expression d'une protéine Met30 entière fusionnée à son extrémité carboxy-terminale, avec la protéine GFP. La protéine hybride Met30-GFP est exprimée sous le contrôle du promoteur *MET30.* Ce plasmide peut se répliquer de façon autonome chez la levure.

### Exemple 6 : Construction des plasmides pYi39XyLex, pYi44XyLex, pLexM4-7, pLexM4Δ239, pM16Z1 et pM16Xyl1 permettant de visualiser l'activité transcriptionnelle des gènes MET de levure.

### 6.1. Construction du plasmide pYi39XyLex :

L'intégration de ce plasmide dans le génome de la levure au locus *TRP1* peut être dirigée en linéarisant ce plasmide par l'enzyme *Stu*1 et en transformant, par le plasmide ainsi linéarisé, une souche de levure portant une mutation ponctuelle du gène *trpl.*

### 6.1.1. construction du plasmide pXylex :

Le fragment d'ADN contenant le promoteur du gène *GAL*1 dont les séquences activatrices de la transcription ont été remplacées par des sites de fixation de la protéine LexA de *E*. *coli,* dont la séquence est celle déposée à la banque EMBL, sous l'identificateur « ECLEXA1 », sous le n° J01643, a été amplifié par PCR à partir du plasmide pSH18-34 (Hanes S. D. et al., (1989), Cell, 57, 1275-1293) en utilisant les oligonucléotides « olGAL1» de séquence SEQ ID N° 12 5' GCCAAGCTTCTCCTTGACGTTAAAGTA 3' et «olGAL10» de séquence SEQ ID N° 13 5' GCCGGATCCTTTGTAACTGAGCTGTCA 3'. L'ADN amplifié à été digéré par les enzymes de restriction *Bam*HI et *HindIII* et inséré dans le vecteur pEMBLYe31-X (Jacquemin-Faure I. et al., (1994), Mol. Gen. Genet., 244, 519-529) préalablement digéré par les enzymes *BamHI* et *Hind*III, produisant le vecteur pXylex.

### 6.1.2. construction du plasmide pYi39 :

Le vecteur pFL39 a été digéré par l'enzyme *Cla*I et a été religaturé sur lui même et un plasmide ayant perdu le fragment *Cla*I*-Cla*I contenant l'origine de réplication ars-cen originale a été sélectionné, produisant le vecteur pYi39.

### 6.1.3. construction du plasmide pYi39Xylex :

Le fragment d'ADN *Bam*HI*-Ps*tI du vecteur pXylex préparé précédemment et contenant le gène *XylE* dont la séquence est celle déposée à la banque EMBL, sous l'identificateur « PPXYLE », sous le n° V01161 précédé de la région promoteur *GAL1-LexA* a été isolé et inséré dans le plasmide pYi39 préalablement digéré par les enzymes *Bam*HI et *Pst*I*,* produisant le plasmide pYi39Xyl-Lex.

### 6.2. Construction du plasmide pYi44Xylex :

### 6.2.1. construction du plasmide pYi44 :

Le vecteur pFL44 a été digéré par l'enzyme ClaI et a été religaturé sur lui même et un plasmide ayant perdu le fragment ClaI-ClaI contenant l'origine de réplication 2µm originale a été sélectionné, produisant le vecteur pYi44.

### 6.2.2. construction du plasmide pYi44Xylex

Le fragment d'ADN *Bam*HI*-Pst*I du vecteur pXylex préparé selon le mode opératoire décrit au point 6.1.1 et contenant le gène *XylE* précédé de la région promoteur *GALI-LexA* a été isolé et inséré dans le plasmide pYi44 préalablement digéré par les enzymes *Bam*HI et *Pst*I*,* produisant le plasmide pY144Xyl-Lex.

### 6.3. Construction du plasmide pLexM4-7 :

### 6.3.1. utilisation :

Il permet l'expression chez la levure S. *cerevisiae* de la protéine Met4 comprenant à son extrémité amino terminale, les 202 résidus de la protéine LexA d*'E.coli.* La protéine hybride ainsi synthétisée est capable de se lier à des régions d'ADN comprenant des opérateurs LexA.

### 6.3.2. construction :

Le fragment d'ADN *EcoRI-BamH*I du vecteur pLexM4-1 codant pour les acides aminés 15 à 666 de la protéine Met4 a été cloné dans le plasmide pBTM116 (Margottin M. et al., (1998), Molecular Cell, 1, 565-574) préalablement digéré par les enzymes *Eco*RI et *Bam*HI*,* produisant le plasmide pLexM4-7. Ce plasmide peut se répliquer de façon autonome chez la levure.

### 6.4. Construction du plasmide pLexM4Δ239

### 6.4.1. utilisation :

Il permet l'expression chez la levure *Saccharomyces cerevisiae* d'un dérivé de la protéine Met4 amputé de ses résidus 212 à 231 comprenant à son extrémité amino terminale, les 202 residus de la protéine LexA d*'E.coli.* La protéine hybride ainsi synthétisée est capable de se lier à des régions d'ADN comprenant des opérateurs LexA.

### 6.4.2. construction:

Le fragment d'ADN *Eco*RI*-Bam*H1 du vecteur pLexM4Δ30 codant pour un dérivé comprenant les acides aminés 15 à 211 et 232 à 666 de la protéine Met4 a été cloné dans le plasmide pBTM 116 (Margottin *M. et al.,* (1998), référence citée) préalablement digéré par les enzymes *Eco*RI *et Bam*HI*,* produisant le plasmide pLexM40239. Ce plasmide peut se répliquer de façon autonome chez la levure.

### 6.5. Construction du plasmide pM16Z1:

Le fragment d'ADN contenant le promoteur du gène *MET16* (comprenant les nucléotides -535 à +3, numérotés à partir du codon d'initiation du gène *MET16*) a été amplifié par PCR à partir du plasmide pM16-1 (Hanes S. D. *et al.,* (1989), référence citée) en utilisant les oligonucléotides «M16OL2» de séquence SEQ ID N° 14 5' CAACGAAGGATCCAATAATCGAAGCC 3' et «M16OL4» de séquence SEQ ID N° 15 5' GGGGAATTCCTTCATTTTATGAGTTGCT 3'. L'ADN amplifié à été digéré par les enzymes de restriction *Bam*HI et *Eco*RI et inséré dans le vecteur Yep356R (Myers A. M. et al. (1986), Gene, 45, 299-310) préalablement digéré par les enzymes *Bam*HI et *Eco*RII, produisant le vecteur pM16Z1. Ce plasmide peut se répliquer de façon autonome chez la levure et permet l'expression de la β-galactosidase de *E. coli* sous le contrôle du promoteur *MET16.*

### 6.6. Construction du plasmide pM16Xyl1 :

Le fragment d'ADN contenant le promoteur du gène *MET16* (comprenant les nucléotides -535 à -1, numérotés à partir du codon d'initiation du gène *MET16*) a été amplifié par PCR à partir du plasmide pM16-1 (Thomas D. et al. (1990), J. Biol. Chem., 265, 15518-15524) en utilisant les oligonucléotides «M16OL5» de séquence SEQ ID N° 16 5' CAACGAAGCTTTCAATAATCGAAGCACTTGG 3' et «M16OL6» de séquence SEQ ID N° 17 5' TTTATGAGAAGCTTTGGGTTGATACCTTTGC 3'. L'ADN amplifié a été digéré par l'enzyme de restriction *Hind*III et inséré dans le vecteur pUC9-LEU2-X (Jacquemin-Faure I. *et al.,* (1994), référence citée) produisant le plasmide pM16Xy11. L'intégration de ce plasmide dans le génome de la levure au locus *LEU2,* peut être dirigée en linéarisant ce plasmide par l'enzyme *Asp*718 et en transformant, par le plasmide ainsi linéarisé, une souche de levure portant une mutation ponctuelle du gène *leu2.* Ainsi ce plasmide permet-il d'obtenir une souche de levure modifiée, exprimant de façon stable la catéchol oxydase de *P. putida,* placée sous le contrôle du gène *MET16.*

### ExempLe 7 : Souches de levure exprimant une protéine hybride GFP-Met4.

Ces souches portent au niveau du locus *URA3* (chromosome V, bras gauche), un gène artificiel permettant l'expression d'une protéine Met4, modifiée ou non, sous le contrôle du promoteur du gène *GAL1.*

L'intégration correcte au locus *URA3* des fusions GAL-Met4-GFP a été vérifiée par des techniques classiques de biologie moléculaire et de génétique.

Les souches qui ont été préparées sont rassemblées dans le Tableau I, ci-après.

**TABLEAU I**

| Nom de la souche de levure | Plasmide utilisé | Caractéristiques de la souche (génotype) |
|---|---|---|
| C300 | pGal306GFPMet4 | *Mata, his3, leu2, trp1, ura3::gall-GFP-Met4::URA3* |
| C301 | pGal306GFPMet4 | *Mata, his3, leu2, trp1, met4::TRP1, ura3::gall-GFP-Met4::URA3* |
| C302 | pGa1306GFPMet4 | *Matα, his3, leu2, trp1, met30-2, tira3::gall-GFP-Met4::URA3* |
| C304 | pGal306GFPMet4Δ12 | *Matα, his3, leu2, trp1, met4:: TRP1, ura3: : gall -GFP-Met4Δ12: : URA3* |
| C305 | pGal306GFPMet4Δ30 | *Matα, his3, leu2, trp1, met4::TRP1, ura3::gall-GFP-Met4Δ30:: URA3* |
| C307 | pGal306GFPMet4Δ37 | *Matα, his3, leu2, trp1, met4::TRP1, ura3::gall-GFP-Met4Δ37:: URA3* |
| C312 | pGal306GFPMet4 | *Mata, his3, leu2, trp1, ura3::gall-GFP-Met4:: URA3* |
| C319 | pGal306GFPMet4 | *Mata, his3, leu2, trp1, cdc34-2, ura3::gall-GFP-Met4::URA3* |
| C323 | pGal306GFPMet4 | *Matα his3, leu2, trp1, cdc53-1, ura3::gall-GFP-Met4::URA3* |
| C327 | pGal306GFPMet4 | *Matα, his3, leu2, trp1, skp1-11, ura3:: gall-GFP-Met4::URA3* |

### 7.1. Préparation des souches à partir de pGa1306GFPMet4 :

L'intégration de ce plasmide dans le génome de la levure au locus *URA3* peut être dirigée en linéarisant ce plasmide par l'enzyme *Stu*I et en transformant, par le plasmide ainsi linéarisé, une souche de levure portant une mutation ponctuelle du gène *ura3.* La fusion GFP-Met4 ainsi intégrée au locus chromosomique *URA3* est exprimée sous le contrôle du promoteur *GAL1.*

### 7.2. Préparation des souches à partir de pGal306GFPMet4Δ12 :

Les souches sont transformées selon le mode opératoire décrit au point 7.1. à partir du plasmide pGa1306GFPMet4Δ12.

### 7.3. Préparation des souches à partir de pGa1306GFPMet4Δ30 :

Les souches sont transformées selon le mode opératoire décrit au point 7.1. à partir du plasmide pGal306GFPMet4Δ30.

### 7.4. Préparation des souches à partir de pGal306GFPMet4Δ37 :

Les souches sont transformées selon le mode opératoire décrit au point 7.1. à partir du plasmide pGal306GFPMet4Δ37.

### Exemple 8 : Souches de levure portant le gène XyLE codant la catéchol oxydase sous le contrôle d'opérateurs LexA.

### 8.1. Préparation des souches :

Ces souches portent au niveau du locus *URA3* (chromosome V, bras gauche) ou au niveau du locus *TRP1* (chromosome IV, bras droit) un gène artificiel comprenant le gène *XylE* de *Pseudomonas putida* placé sous le contrôle d'opérateurs LexA. L'expression de ce gène est dirigée par la protéine hybride LexA-Met4 exprimée à partir du plasmide réplicatif pLexM4-7.

L'intégration correcte au locus *URA3* des fusions GAL-Met4-GFP a été vérifiée per des techniques classiques de biologie moléculaire et de génétique.

Les souches qui ont été préparées sont rassemblées dans le Tableau II, ci-après.

**TABLEAU II**

| Nom de la souche de levure | Plasmide utilisé | Caractéristiques de la souche (génotype) |
|---|---|---|
| C 190 | pYi44Xylex | *Mata, ade2, his3, leu2, trp1,* ura3::LeAₒₚ-XylE::URA3 |
| C 192 | PYi39Xylex | *Mata, ade2, his3, leu2, trp1,* Trpl::LeXAₒₚ-XylE::TRP1 |
| C 193 | pYi44Xylex | *Mata, ade2, his3, leu2, trp1, met4::TRP1, ura3::LeAₒₚ-XylE:: URA3* |

### 8.2. Préparation des souches à partir du plasmide pYi44XyLex :

L'intégration de ce plasmide dans le génome de la levure au locus *URA3* peut être dirigée en linéarisant ce plasmide par l'enzyme *Stu1* et en transformant par le plasmide ainsi linéarisé, une souche de levure portant une mutation ponctuelle du gène *ura3* (Orr-Weaver T. L. *et al.* (1983) et Rothstein R. (1991), références citées).

### 8.3. Préparation des souches à partir du plasmide pYi39XyLex

L'intégration de ce plasmide dans le génome de la levure au locus *TRP1* peut être dirigée en linéarisant ce plasmide par l'enzyme *Stu*1 et en transformant par le plasmide ainsi linéarisé, une souche de levure portant une mutation ponctuelle du gène *trp1* (Orr-Weaver T. L*. et al.* (1983) et Rothstein R. (1991), références citées).

### Exemple 9 : Souche de levure portant le gène XylE codant la catéchol oxydase sous le contrôle du promoteur du gène MET25.

### 9.1. Préparation de la souche CC634-2D :

Cette souche est issue du croisement entre la souche CI2-11D (Jacquemin-Faure I. *et al.,* (1994), référence citée) et la souche CD 106 (Thomas D. *et al.,* (1992), référence citée). Elle comporte, intégrée au locus *LEU2* (chromosome III, bras gauche) un gène *XylE* placé sous le contrôle du promoteur du gène *MET25.*

### 9.2. Caractéristiques de la souche CC634-2D :

Le génotype de cette souche est le suivant : *Mata, ade2, his3, trp1, ura3, met4::TRP1, leu2::proMet25-XylE::LEU2.*

### Exemple 10 : Souche de levure portant le gène HIS3 sous le contrôle d'opérateurs LexA.

Ces souches portent au niveau du locus *LYS2* (chromosome II, bras droit) un gène artificiel comprenant le gène *HIS3* de *S. cerevisiae* placé sous le contrôle d'opérateurs LexA. L'expression de ce gène est dirigée par la protéine hybride LexA-Met4 exprimée à partir du plasmide réplicatif pLexM4-7.

### 10.1. Préparation des souches

### 10.1.1. Préparation de la souche CC817-4A :

Cette souche est issue du croisement entre la souche L40 (Hollenberg S. M. et al., (1995), Mol. Cell Biol., 15, 3813-3822) et la souche CC816-1D de génotype Matα, ade2, leu2, lys2, trp1, ura3.

### 10.1.2. Préparation de la souche CY25-1D :

Cette souche est issue du croisement entre la souche CC817-4A préparée précédemment et la souche CD 106 (Thomas *D. et al.,* (1992), référence citée).

### 10.2. Caractéristiques des souches:

### 10.2.1. Caractéristiques de la souche CC817-4A:

Le génotype de cette souche est le suivant : *Mata, ade2, his3, leu2, trp1, ura3, lys2::LexAop-HIS3::LYS2*

### 10.2.2. Caractéristiques de la souche CY25-1D:

Le génotype de cette souche est le suivant : *Mata, ade2, his3, leu2, trp1, ura3 mel32::URA3, lys2::LexAop-HIS3::LYS2*

### Exemple 11 : Souche de levure portant le gène LacZ sous le contrôle d'opérateurs LexA.

Ces souches portent au niveau du locus *URA3* (chromosome V, bras gauche) un gène artificiel comprenant le gène *LacZ d'E. coli* placé sous le contrôle d'opérateurs LexA. L'expression de ce gène est dirigée par la protéine hybride LexA-Met4 exprimée à partir du plasmide réplicatif pLexM4-7.

### 11.1. Préparation de la souche CC801-3B

Cette souche est issue du croisement entre la souche L40 (Hollenberg S. *M. et al.,* (1995), référence citée) et la souche CD 106 (Thomas D*. et al.,* (1992), référence citée).

### 11.2. Caractéristiques de la souche CC801-3B :

Le génotype de cette souche est le suivant : *Mata, ade2, his3, leu2, trp1, ura3 met4::TRP1, ura3::LexAop-LacZ:: URA3*

### Exemple 12: Détermination du niveau d'expression et de la stabilité des protéines exprimées à partir des séquences hybrides contenues dans les plasmides selon l'invention.

### 12.1. Mode opératoire :

### 12.1.1. Détermination de la stabilité des protéines hybrides:

### a) Visualisation par le marqueur antigénigue hémagglutinine (Ha) :

Des cellules de levure comportant un plasmide codant pour les protéines marquées par Ha, sous le contrôle du promoteur *GAL*1*,* sont cultivées dans un milieu contenant du raffinose.

L'induction de la protéine hybride, exprimée sous le contrôle du promoteur *GAL*1*,* est réalisée pendant 2 à 10 heures, en transférant les cellules dans un milieu contenant 2 à 5 % de galactose.

Des prélèvements sont effectués à 0, 5, 10, 20, 40, 60 et 80 minutes après l'addition de glucose et éventuellement de méthionine, à une concentration répressive comprise entre 0,05 mM et 25 mM.

La stabilité des protéines hybrides est mesurée par réaction avec des anticorps anti-Ha, selon des techniques classiques.

### b) Visualisation par la protéine GFP :

Des cellules de levure comportant un plasmide codant pour les protéines marquées par la protéine GFP, sous le contrôle du promoteur *GAL*1*,* sont cultivées dans un milieu contenant du raffinose et l'induction de la protéine hybride est réalisée selon le mode opératoire décrit précédemment.

La mesure de la fluorescence est réalisée après 20 minutes d'incubation en présence ou en absence de concentrations répressives de méthionine.

### c) Visualisation par la catéchol-oxydase :

L'activité du gène rapporteur LexAopXyle est mesurée dans une souche de levure (C190) exprimant la protéine hybride codée par le plamide pLexMet4-7 et cultivée dans les conditions décrites précédemment.

L'activité est mesurée par une technique de visualisation basée sur la mesure de la catéchol-oxydase selon des techniques classiques.

### 12.1.2. Mesure des ARNs totaux :

Elle est réalisée par des techniques classiques connues de l'homme du métier.

### 12.2. Résultats :

### 12.2.1 Stabilité des protéines hybrides :

### a) Visualisation par le marqueur antigénigue hémaglutinine (Ha)

Ils sont rassemblés dans les figures 1 et 3B.

### * Expression de la protéine hybride Ha-Met4

La protéine Ha-Met4 a une demi-vie de l'ordre de 20 minutes en l'absence de méthionine (figure 1A, - Met) et une demi-vie de l'ordre de 5 minutes dans des conditions répressives, c'est à dire en présence de méthionine (figure 1A, + Met).

La dégradation de la protéine Ha-Met4 est presque complète dans des conditions répressives au bout de 20 minutes (figure 1A, + Met).

### * Expression de la protéine hybride Ha-Met28

La protéine Ha-Met28 a une demi-vie de l'ordre de 20 minutes en l'absence de méthionine (Figure 1A, - Met).

Les conditions répressives ne modifient pas la demi-vie de la protéine Ha-Met28 (Figure 1A, + Met).

### * Expression des protéines hybrides Ha-Met30 et Ha-Met30ΔF :

La protéine Ha-Met30 a une demi-vie de l'ordre de 20 minutes en l'absence de méthionine (figure 3B, - Met).

Les conditions répressives ne modifient pas la demi-vie de la protéine hybride Ha-Met30 (figure 3B, + Met).

La protéine hybride Ha-Met30ΔF apparaît moins stable que la protéine hybride Ha-Met30, mais des conditions répressives ne diminuent pas la stabilité de cette protéine hybride.

### b) Visualisation par des protéine hybrides comprenant GFP

Les résultats sont illustrés dans les figures 2 et 3B.

### * Localisation de la protéine hybride GFP-Met4

La protéine hybride GFP-Met4 est localisée dans le noyau quand les cellules sont cultivées en l'absence de méthionine.

En revanche, dans des conditions répressives, c'est à dire en présence de méthionine, cette localisation n'est plus observée (figure 2A).

Ces résultats sont en accord avec une dégradation rapide de la protéine hybride dans des conditions répressives.

### * Localisation de la protéine hybride GFP-Met28

La protéine hybride GFP-Met28 est toujours présente dans le noyau, que ce soit en absence ou en présence de méthionine (figure 2B).

### * Localisation de la protéine hybride GFP-Met30

La protéine hybride GFP-Met30 est toujours présente dans le noyau, que ce soit en absence ou en présence de méthionine (figure 3A).

### 12.2.2. Expression des ARN totaux

Les résultats sont illustrés dans les figures 1B et 1C

Les taux de Met4 et de Met28 permettent l'activation de la transcription des gènes cibles.

Ni l'expression de *MET4,* ni celle de *MET28,* sous le contrôle du promoteur *GAL*1*,* ne modifie la répression induite par l'addition de méthionine.

### 12.2.3. Visualisation par la catéchol-oxydase

Les résultats sont illustrés à la figure 4.

En l'absence de méthionine, les cellules sont colorées en jaune, preuve que la protéine est exprimée.

En présence de méthionine, les cellules sont blanches du fait de la répression.

## Revendications

1. Procédé de criblage cellulaire de composés aptes à agir sur les pathologies liées au dysfonctionnement de la cascade ubiquitine-protéasome chez l'Homme, **caractérisé en ce qu'**il comprend les étapes suivantes :
(i) la mise en contact du produit à tester avec une souche de levure modifiée, comportant (a) une séquence hybride, comprenant une séquence codant pour une protéine Met4, sous sa forme sauvage ou mutée, fusionnée en phase avec au moins une séquence codant pour un marqueur approprié, ladite séquence hybride étant exprimée sous le contrôle d'un promoteur, actif chez la levure et éventuellement (b) un système transcriptionnel rapporteur, constitué par un gène rapporteur placé sous le contrôle d'une séquence opératrice appropriée ou d'un promoteur de levure approprié,
(ii) l'addition de méthionine, à des concentrations répressives, comprises entre 0,03 mM et 20 mM, ou non répressives, et
(iii) la détermination du niveau d'expression et de la stabilité de la protéine exprimée à partir de la séquence hybride, soit par visualisation et/ou quantification, soit par détermination de l'activité du gène rapporteur.

2. Procédé selon la revendication 1, **caractérisé en ce qu'**il comprend, en parallèle, les étapes suivantes :
(iv) la mise en contact du produit à tester avec une souche de levure modifiée, comportant une séquence hybride, comprenant une séquence codant pour une protéine Met30, sous sa forme sauvage ou mutée, fusionnée en phase avec au moins une séquence codant pour un marqueur approprié, ladite séquence hybride étant exprimée sous le contrôle d'un promoteur, actif chez la levure,
(v) l'addition de méthionine, à des concentrations répressives, comprises entre 0,03 mM et 20 mM, ou non répressives, et
(vi) la détermination du niveau d'expression et de la stabilité de la protéine exprimée à partir de la séquence hybride, soit par visualisation et/ou quantification, soit par détermination de l'activité du gène rapporteur

3. Procédé selon l'une quelconque des revendications 1 et 2, **caractérisé en ce qu'**il comprend en outre, un moyen supplémentaire mettant en oeuvre un système cellulaire de contrôle, ledit moyen comprenant les étapes suivantes :
(vii) la mise en contact du produit à tester avec une souche de levure modifiée, comportant une séquence hybride, comprenant une séquence codant pour une protéine Met28, sous sa forme sauvage ou mutée, fusionnée en phase avec au moins une séquence codant pour un marqueur approprié, ladite séquence hybride étant exprimée sous le contrôle d'un promoteur, actif chez la levure,
(viii) l'addition de méthionine, à des concentrations répressives, comprises entre 0,03 mM et 20 mM, ou non répressives, et
(ix) la détermination du niveau d'expression et de la stabilité de la protéine exprimée à partir de la séquence hybride, soit par visualisation et/ou quantification, soit par détermination de l'activité du gène rapporteur.

4. Procédé selon l'une quelconque des revendications 1 à 3, **caractérisé en ce qu'**ils comprend en outre un moyen supplémentaire mettant en oeuvre un système acellulaire de contrôle qui repose sur la mesure des taux de transcription des séquences hybrides et des gènes métaboliques *MET16* et *MET25,* ledit moyen comprenant les étapes suivantes :
(x) l'extraction des ARNs totaux des souches modifiées utilisées soit à l'étape (i), soit à l'étape (iv), soit à l'étape (vii) et
(xi) la mesure des taux de transcription de la séquence hybride et celle des gènes métaboliques *MET16* et *MET25.*

5. Procédé selon l'une quelconque des revendications 1 à 4, **caractérisé en ce que** le marqueur utilisé pour la construction de la séquence hybride est choisi dans le groupe constitué par : les peptides antigéniques, les protéines à fluorescence intrinsèque, les protéines à activité enzymatique mesurable et les facteurs de liaison à l'ADN.

6. Procédé selon l'une quelconque des revendications 1 à 5, **caractérisé en ce que** le promoteur permettant l'expression de la protéine hybride est choisi dans le groupe constitué par les promoteurs inductibles actifs chez S. *cerevisiae* et les promoteurs constitutifs.

7. Procédé selon l'une quelconque des revendications 1 à 6, **caractérisé en ce que** le marqueur utilisé pour la construction de ladite séquence hybride est le facteur de liaison à l'ADN LexA, et ledit gène rapporteur est placé sous le contrôle d'opérateurs LexA.

8. Procédé de criblage cellulaire de composés aptes à agir sur les pathologies liées au dysfonctionnement de la cascade ubiquitine-protéasome chez l'Homme, **caractérisé en ce qu'**il comprend les étapes suivantes :
(xii) la mise en contact du produit à tester avec une souche de levure modifiée, comportant un système transcriptionnel rapporteur, constitué par un gène rapporteur placé sous le contrôle d'un promoteur activé par le facteur de transcription Met4,
(xiii) l'addition de méthionine, à des concentrations répressives, comprises entre 0,03 mM et 20 mM, ou non répressives, et
(xiv) la détermination de l'activité du gène rapporteur.

9. Procédé selon l'une quelconque des revendications 1 à 8, **caractérisé en ce que** le gène rapporteur présent dans le système rapporteur transcriptionnel est choisi dans le groupe constitué par les gènes rapporteurs dont l'activité est visualisable par une méthode colorimétrique et les gènes métaboliques, dont l'activité est mesurable par un test de croissance.

10. Procédé selon l'une quelconque des revendications 1 à 9, **caractérisé en ce que** ledit gène rapporteur est placé sous le contrôle du promoteur d'un gène *MET.*

11. Procédé selon la revendication 10, **caractérisé en ce que** ledit gène *MET* est *MET3, MET10, MET16, MET25* ou *MET28.*

## Claims

1. Process for cellular screening of compounds capable of acting on pathologies connected with a disfunction of the ubiquitin-proteasome cascade in human, **characterised in that** it comprises the following steps:
(i) contacting the product which is to be tested with a modified yeast strain, comprising (a) a hybrid sequence comprising a sequence coding for a Met4 protein, in its wild-type or mutated form, fused in phase with at least one sequence coding for a suitable marker, said hybrid sequence being expressed under the control of a promoter which is active in yeast and optionally (b) a reporter transcription system consisting of a reporter gene placed under the control of a suitable operating sequence or a suitable yeast promoter,
(ii) adding methionine, at inhibitory concentrations between 0.03 mM and 20 mM, or at non-inhibitory concentrations, and
(iii) determining the expression level and stability of the protein expressed from the hybrid sequence either by visualisation and/or quantification, or by determining the activity of the reporter gene.

2. Process according to claim 1, **characterised in that** it comprises the following steps carried out in parallel:
(iv) contacting the product which is to be tested with a modified yeast strain, comprising a hybrid sequence having a sequence coding for a Met30 protein, in its wild-type or mutated form, fused in phase with at least one sequence coding for a suitable marker, said hybrid sequence being expressed under the control of a promoter which is active in yeast,
(v) adding methionine at inhibitory concentrations of between 0.03 mM and 20 mM or at non-inhibitory concentrations, and
(vi) determining the expression level and stability of the protein expressed from the hybrid sequence, either by visualisation and/or quantification,or by determining the activity of the reporter gene.

3. Process according to any one of claims 1 and 2, **characterised in that** it further comprises a supplementary means using a cell control system, said means comprising the following steps:
(vii) contacting the product which is to be tested with a modified yeast strain comprising a hybrid sequence, comprising a sequence coding for a Met28 protein, in its wild-type or mutated form, fused in phase with at least one sequence coding for a suitable marker, said hybrid sequence being expressed under the control of a promoter which is active in yeast,
(viii) adding methionine at inhibitory concentrations of between 0.03 mM and 20 mM or at non-inhibitory concentrations, and
(ix) determining the expression level and stability of the protein expressed from the hybrid sequence, either by visualisation and/or quantification, or by determining the activity of the reporter gene.

4. Process according to any one of claims 1 to 3, **characterised in that** it further comprises a supplementary means using a non-cellular control system based on measuring the transcription levels of the hybrid sequence and of the metabolic genes MET16 and MET25, said means comprising the following steps:
(x) extracting the total RNAs from the modified strains used either in step (i) or in step (iv) or in step (vii), and
(xi) measuring the transcription levels of the hybrid sequence and that of the metabolic genes MET16 and MET25.

5. Process according to any one of claims 1 to 4, **characterised in that** the marker used to construct the hybrid sequence is selected from the group consisting of -: the antigenic peptides, the intrinsically fluorescent proteins, the proteins with a measurable enzymatic activity and the DNA binding factors.

6. Process according to any one of claims 1 to 5, **characterised in that** the promoter that allows expression of the hybrid protein is selected from the group consisting of the inducible promoters which are active in *S. cerevisiae* and the constitutive promoters.

7. Process according to any one of claims 1 to 6, **characterised in that** the marker used to construct said hybrid sequence is the DNA binding factor LexA, and said reporter gene is placed under the control of LexA operators.

8. Process for the cellular screening of compounds capable of acting on pathologies linked with the disfunction of the ubiquitin-proteasome cascade in human, **characterised in that** it comprises the following steps:
(xii) contacting the product which is to be tested with a modified yeast strain, comprising a reporter transcription system, consisting of a reporter gene placed under the control of a promoter activated by the transcription factor Met4,
(xiii) adding methionine at inhibitory concentrations of between 0.03 mM and 20 mM or non-inhibitory concentrations and
(xiv) determining the activity of the reporter gene.

9. Process according to any one of claims 1 to 8, **characterised in that** the reporter gene present in the reporter transcription system is selected from the group consisting of the reporter genes the activity of which can be visualised by a colorimetric method and the metabolic genes the activity of which can be measured by a growth test.

10. Process according to any one of claims 1 to 9, **characterised in that** the reporter gene is placed under the control of the promoter of a MET gene.

11. Process according to claim 10, **characterised in that** the MET gene is MET3, MET10, MET16, MET25 or MET28.

## Patentansprüche

1. Zelluläres Screening-Verfahren von Mischungen, die geeignet sind auf die mit Funktionsstörungen der Ubiquitin-Proteasomkaskade beim Menschen verbundenen Krankheiten zu wirken, **dadurch gekennzeichnet, dass** es die folgenden Schritte umfasst:
(i) in Kontakt bringen der zu untersuchenden Substanz mit einem veränderten Hefestamm, der (a) eine Hybridsequenz umfasst, die eine codierende Sequenz für ein Met4-Protein in seiner mutierten oder Wildtypform enthält und phasengleich mit wenigstens einer codierenden Sequenz für einen geeigneten Marker verbunden ist, wobei die Hybridsequenz unter der Kontrolle eines bei der Hefe aktiven Promotors exprimiert wird, und der unter Umständen (b), ein Reporter-Transkriptionssystem umfasst, das aus einem unter die Kontrolle einer geeigneten operativen Sequenz oder eines geeigneten Hefepromotors gestelltem Reportergen besteht,
(ii) Zugabe von Methionin in repressiven Konzentrationen, einen Bereich von 0,03 M und 20 M umfassend, oder nicht repressiven Konzentrationen, und
(iii) Bestimmung des Expressionsgrades und der Stabilität des exprimierten Proteins ausgehend von der Hybridsequenz, sei es durch Visualisierung und/oder Quantifizierung, sei es durch Bestimmung der Aktivität des Reportergens.

2. Verfahren nach Anspruch 1, **dadurch gekennzeichnet, dass** es parallel laufend folgende Schritte umfasst:
(iv) in Kontakt bringen der zu untersuchenden Substanz mit einem veränderten Hefestamm, der eine Hybridsequenz umfasst, die eine codierende Sequenz für ein Met30-Protein in seiner mutierten oder Wildtypform enthält und phasengleich mit wenigstens einer codierenden Sequenz für einen geeigneten Marker verbunden ist, wobei die Hybridsequenz unter der Kontrolle eines bei der Hefe aktiven Promoters exprimiert wird,
(v) Zugabe von Methionin in repressiven Konzentrationen, einen Bereich zwischen 0,03 Mm und 20 Mm umfassend, oder nicht repressiven Konzentrationen, und
(vi) Bestimmung des Expressionsgrades und der Stabilität des exprimierten Proteins ausgehend von der Hybridsequenz, sei es durch Visualisierung und/oder Quantifizierung, sei es durch Bestimmung der Aktivität des Reportergens.

3. Verfahren nach einem der Ansprüche 1 und 2, **dadurch gekennzeichnet, dass** es darüber hinaus einen zusätzlichen Weg umfasst, welcher ein zelluläres Kontrollsystem einleitet, wobei der zusätzliche Weg die folgenden Schritte umfasst:
(vii) in Kontakt bringen der zu untersuchenden Substanz mit einem veränderten Hefestamm, der eine Hybridsequenz umfasst, die eine codierende Sequenz für ein Met28-Protein in seiner mutierten oder Wildtypform enthält und phasengleich mit wenigstens einer codierenden Sequenz für einen geeigneten Marker verbunden ist, wobei die Hybridsequenz unter der Kontrolle eines bei der Hefe aktiven Promotors exprimiert wird,
(viii) Zugabe von Methionin in repressiven Konzentrationen, einen Bereich von 0,03Mm und 20Mm umfassend oder nicht repressiven Konzentrationen, und
(ix) Bestimmung des Expressionsgrades und der Stabilität des exprimierten Proteins ausgehend von der Hybridsequenz, sei es durch Visualisierung und/oder Quantifizierung, sei es durch Bestimmung der Aktivität des Reportergens.

4. Verfahren nach einem der Ansprüche 1 bis 3, **dadurch gekennzeichnet, dass** es darüber hinaus einen zusätzlichen Weg umfasst, welcher ein nichtzelluläres Kontrollsystem einleitet, welches auf der Messung der Transkriptionsraten der Hybridsequenzen und der metabolischen Gene *MET16* und *MET25* beruht, wobei der Weg die folgenden Schritte umfasst:
(x) Extraktion der gesamten DNA der verwendeten modifizierten Stämme, sei es aus Schritt (i), aus Schritt (iv), aus Schritt (vii) und
(xi) Messen der Transkriptionsrate der Hybridsequenz und der der metabolischen Gene *MET16* und *MET25.*

5. Verfahren nach einem der Ansprüche 1 bis 4, **dadurch gekennzeichnet, dass** der für die Konstruktion der Hybridsequenz verwendete Marker ausgewählt ist aus der Gruppe, bestehend aus Antigenpeptiden, Proteinen mit intrinsischer Fluoreszenz, Proteinen mit messbarer enzymatischer Aktivität und DNA-Bindungsfaktoren.

6. Verfahren nach einem der Ansprüche 1 bis 5, **dadurch gekennzeichnet, dass** der die Expression des Hybridproteins ermöglichende Promotor ausgewählt ist aus der Gruppe, bestehend aus aktiven induzierbaren Promotoren bei S. *cerevisiae* und konstitutiven Promotoren.

7. Verfahren nach einem der Ansprüche 1 bis 6, **dadurch gekennzeichnet, dass** der für die Konstruktion der Hybridsequenz verwendete Marker der DNA-Bindungsfaktor LexA ist, und das Reportergen unter die Kontrolle der LexA-Operatoren gestellt ist.

8. Zelluläres Screening-Verfahren von Mischungen, die geeignet sind auf die mit Funktionsstörungen der Ubiquitin-Proteasomkaskade beim Menschen verbunden Krankheiten zu wirken, **dadurch gekennzeichnet, dass** es die folgenden Schritte umfasst:
(xii) in Kontakt bringen der zu untersuchenden Substanz mit einem veränderten Hefestamm, der ein Reporter-Transkriptionssystem umfasst, dass aus einem unter die Kontrolle eines Promotors, der durch den Transkriptionsfaktor Met4 aktiviert wird, gestellten Reportergen besteht,
(xiii) Zugabe von Methionin in repressiven Konzentrationen, einen Bereich von 0,03Mm und 20Mm umfassend oder nicht repressiven Konzentrationen, und
(xiv) Bestimmung der Aktivität des Reportergens.

9. Verfahren nach einem der Ansprüche 1 bis 8, **dadurch gekennzeichnet, dass** das in dem Reporter-Transkriptionssystem vorliegende Reportergen ausgewählt ist aus der Gruppe, bestehend aus den Reportergenen, deren Aktivität durch ein colorimetrisches Verfahren visualisierbar ist und metabolischen Genen, deren Aktivität durch eine Untersuchung der Zunahme messbar ist.

10. Verfahren nach einem der Ansprüche 1 bis 9, **dadurch gekennzeichnet, dass** das Reportergen unter die Kontrolle des Promotors eines *MET* Gens gestellt wird.

11. Verfahren nach Anspruch 10, **dadurch gekennzeichnet, dass** das *MET-* Gen *MET3, MET10, MET16, MET25* oder *MET28* ist.


### LISTE DE SEQUENCES

<110> CENTRE NATIONAL DE LA RECHERCHE SCIENTIFIQUE-CNRS
   THOMAS Dominique
   BARBEY Regine
   ROUILLON Astrid
   KERJAN Yolande
<120> PROCEDES DE CRIBLAGE CELLULAIRE DE COMPOSES APTES A MODULER L'ACTIVITE DES COMPLEXES UBIQUITINE-LIGASES SCF ET LEURS APPLICATIONS
<130> s644FR45
<140>
   <141>
<160> 17
<170> PatentIn Ver. 2.1
<210> 1
   <211> 23
   <212> ADN
   <213> Séquence artificielle
<220>
   <223> Description de la séquence artificielle: amorce
<400> 1
   caaagaagct taataatcat att 23
<210> 2
   <211> 19
   <212> ADN
   <213> Séquence artificielle
<220>
   <223> Description de la séquence artificielle: amorce
<400> 2
   ttgaccaact ggctgagcc 19
<210> 3
   <211> 161
   <212> ADN
   <213> Séquence artificielle
<220>
   <223> Description de la séquence artificielle: amorce
<400> 3
   gtaccatgta cccatacgac gttccagact acgcttcttt gggtggttct agcccaagct 60
   cagagctcca ccgcggtggc ggccgcatct tttacccata cgatgttcct gactatgcgg 120
   gctatcccta tgacgtcccg gactatgcag gatccacgta t                     161
<210> 4
   <211> 28
   <212> ADN
   <213> Séquence artificielle
<220>
   <223> Description de la séquence artificielle: amorce
<400> 4
   acgcgaattc atgtctaaag gtgaatta 28
<210> 5
   <211> 28
   <212> ADN
   <213> Séquence artificielle
<220>
   <223> Description de la séquence artificielle: amorce
<400> 5
   acgcgaattc tttgtacaat tcatccat 28
<210> 6
   <211> 62
   <212> ADN
   <213> Séquence artificielle
<220>
   <223> Description de la séquence artificielle: amorce
<400> 6
   tgaaattgtt gaatgacatt aagagacgga acatgggcag gtctaaaggt gaagaattat 60
   tc                                                                62
<210> 7
   <211> 61
   <212> ADN
   <213> Séquence artificielle
<220>
   <223> Description de la séquence artificielle: amorce
<400> 7
   agtctgtgga atataaacgg tccttgatca atgcggagtg ttatttgtac aattcatcca 60
   t 61
<210> 8
   <211> 60
   <212> ADN
   <213> Séquence artificielle
<220>
   <223> Description de la séquence artificielle: amorce
<400> 8
   gggtgcgtaa aaatgtacaa attcgatctc aatgattcta aaggtgaaga attattcact 60
<210> 9
   <211> 52
   <212> ADN
   <213> Séquence artificielle
<220>
   <223> Description de la séquence artificielle: amorce
<400> 9
   tagggcgaat tggagctcca ccgcggtggc ttatttgtac aattcatccc at 52
<210> 10
   <211> 60
   <212> ADN
   <213> Séquence artificielle
<220>
   <223> Description de la séquence artificielle: amorce
<400> 10
   gggtgcgtaa aaatgtacaa attcgatctc aatgattcta aaggtgaaga attattcact 60
<210> 11
   <211> 61
   <212> ADN
   <213> Séquence artificielle
<220>
   <223> Description de la séquence artificielle: amorce
<400> 11
   gagtaatagc atctaatggt caagagtttt atcgagacga ttatttgtac aattcatcca 60
   t 61
<210> 12
   <211> 27
   <212> ADN
   <213> Séquence artificielle
<220>
   <223> Description de la séquence artificielle: amorce
<400> 12
   gccaagcttc tccttgacgt taaagta 27
<210> 13
   <211> 27
   <212> ADN
   <213> Séquence artificielle
<220>
   <223> Description de la séquence artificielle: amorce
<400> 13
   gccggatcct ttgtaactga gctgtca 27
<210> 14
   <211> 26
   <212> ADN
   <213> Séquence artificielle
<220>
   <223> Description de la séquence artificielle: amorce
<400> 14
   caacgaagga tccaataatc gaagcc 26
<210> 15
   <211> 28
   <212> ADN
   <213> Séquence artificielle
<220>
   <223> Description de la séquence artificielle: amorce
<400> 15
   ggggaattcc ttcattttat gagttgct 28
<210> 16
   <211> 31
   <212> ADN
   <213> Séquence artificielle
<220>
   <223> Description de la séquence artificielle: amorce
<400> 16
   caacgaagct ttcaataatc gaagcacttg g 31
<210> 17
   <211> 31
   <212> ADN
   <213> Séquence artificielle
<220>
   <223> Description de la séquence artificielle: amorce
<400> 17
   tttatgagaa gctttgggtt gatacctttg c 31